# EUROPEAN PATENT SPECIFICATION

(11) **EP 4 072 552 B1**
(45) Date of publication and mention of the grant of the patent: **18.12.2024**
(21) Application number: 20828088.3
(22) Date of filing: 11.12.2020
(51) Int. Cl.: A61K 31/4745, A61K 9/00, A61K 47/34, A61P 35/00, A61K 45/06, A61K 31/47, A61K 31/495

(54) **CHEMOTHERAPEUTIC DRUG IMPLANT**
CHEMOTHERAPEUTIKUMIMPLANTAT
IMPLANT DE MEDICAMENT CHIMIOTHERAPIQUE

(30) Priority: 12.12.2019 GB 201918300
(43) Date of publication of application: 19.10.2022
(73) Proprietor: Extruded Pharmaceuticals Ltd, Erdington Birmingham West Midlands B23 6HP (GB)
(72) Inventor: MCCONVILLE, Christopher, Birmingham West Midlands B23 6HP (GB)
(74) Representative: HGF
(86) International application number: PCT/GB2020/053185
(87) International publication number: WO 2021/116701

(56) References cited:
- EP-A1- 1 985 286
- WO-A1-2014/059022
- WO-A1-2016/030748
- WO-A2-2006/027567

## Description

This invention relates to chemotherapeutic drug implants, and more particularly, to biodegradable chemotherapeutic drug implants comprising irinotecan, or 7-ethyl-10-hydroxy-camptothecin (an active metabolite of irinotecan), or pharmaceutically acceptable salts thereof. The present invention also relates to processes for the preparation of these chemotherapeutic drug implants and to their use in therapy, in particular in treating brain tumours.

### BACKGROUND

The most common malignant primary brain tumour in adults is glioblastoma multiforme (GBM) [Dolecek et al., Neuro. Oncol. (2012), 14, Suppl 5:v1-49.]. GBM accounts for 45.2% of primary malignant brain and CNS tumour gliomas [Ostrom et al., Neuro. Oncol. (2013), 15, 1-56], with an annual incidence rate of 3.19 per 100,000 worldwide [Thakkar et al., Cancer Epidemiol. Biomarkers Prev. (2014), 23, 1985-1996]. It is invasive and one of the most aggressive tumours of the central nervous system, having the histological features of high cellularity, nuclear atypia, microvascular proliferation, brisk mitotic activity and necrosis. Upon initial diagnosis of GBM, the standard treatment is known as the Stupp protocol [Stupp et al. New Engl. J. Med., (2005), 10, 987-96] and involves surgical resection ('debulking') with maximal tumour-free margins, radiotherapy with concomitant temozolomide chemotherapy and additional higher dose temozolomide chemotherapy after each radiotherapy cycle. Even with complete surgical resection of the tumour, combined with this very aggressive treatment, the overall survival of patients remains at just 12-15 months with a 5-year survival of 5% [Ostrom et al., Neuro. Oncol. (2014), 16, 896-913].

Due to GBM being an extremely infiltrative brain cancer, and thus impossible to fully remove surgically, tumour recurrence is almost inevitable and 80 to 90% recur within 2 cm of the resection site [Wang et al., Adv. Drug Deliv. Rev. (2002), 54, 987-1013]. There is no established chemotherapy regimen available to patients who recur and increasing numbers of patients with recurrent GBM are undergoing re-operation to control their disease where conventional second line therapy has failed.

The reason most GBM treatments fail is because they are administered either via the intravenous or oral route. Systemic delivery of chemotherapeutic drugs to the brain is limited by the blood-brain barrier (BBB) and administration of high systemic drug levels is required to achieve the therapeutic levels needed in the brain. Local delivery directly into the resection margin at the time of surgery using an implantable device would allow for the delivery of chemotherapeutic drugs directly to the brain offering a number of advantages such as a lower dose, increased patient tolerance, and reduced side effects due to the avoidance of systemic circulation.

The Gliadel^{®} wafer is a local delivery device, which was approved by the Food and Drug Administration in 1996 for the treatment of recurring GBM. It is a disc-shaped, 200 mg biodegradable wafer containing 3.85% w/w of the chemotherapeutic agent Carmustine. Gliadel^{®} has demonstrated a small but significant benefit in combination with surgery for patients with recurrent gliomas [Westphal et al., Neuro. Oncol. (2003), 5, 79-88]. However, Gliadel^{®} and other similar approaches are limited by the reliance on drug diffusion from the device into the brain parenchyma restricting penetration distances to a few millimetres.

Biodegradable implants are used for local drug delivery as they can be administered relatively easily and designed to control the release of a wide range of therapeutic agents. For instance, Ramachandran *et al* describe the delivery of Temozolomide from a theranostic nano brain-implant for the prolonged and localized treatment of recurrent glioma [Ramachandran et al., Sci Rep. (2017), 7, 43271]. McConville *et al describe* the development of disulfiram-loaded poly lactic glycolic acid (PLGA) millirods for the treatment of GBM administered to the tumour via stereotactic injection [McConville et al., Int. J. Pharm. (2015) 494(1), 73-82]. Lesniak *et al* demonstrated the efficacy of doxorubicin-loaded biodegradable wafers using a GBM rat model [Lesniak et al., Anticancer Res. (2005) 25, 3825-3831], while Zembeko *et al* describe the manufacture of disulfiram-loaded PLGA wafers for the localised treatment of GBM [Zembeko et al., J. Pharm. Sci. (2015) 104(3), 1076-86].

Irinotecan (IRN) is a semi-synthetic pro-drug, whose active metabolite 7-ethyl-10-hydroxycamptothecin, also referred to as SN-38, acts as an inhibitor of the Topoisomerase I group of enzymes. Topoisomerase I enzymes act within the cell to induce temporary cuts within one or both strands of DNA, allowing the DNA to uncoil for transcription and replication. During this process, Topoisomerase I forms a covalent linkage with DNA, allowing it to form a cleavable complex. SN-38 binds to Topoisomerase I in this confirmation, inhibiting the enzymes from re-joining the strands of DNA, causing S-phase specific cell killing [Xu, Ann. Oncol. (2002) 13, 1841-1851**;** Sinha, Drugs. (1995), 49, 11-19; Vrendenburgh et al., Neuro. Oncol. (2009) 11, 80-91].

Currently, IRN is part of the standard treatment regimen for advanced colorectal cancer, when used in combination with 5-fluorouracil (5-FU) and folinic acid. In treating GBM, IRN administered as an intravenous monotherapy had a response rate between 0 and 44%, with progression free survival between 2 to 11 months; in combination with other drugs IRN had a response rate between 13 and 100%, with progression free survival of 3 to 12 months [Vrendenburgh et al., Neuro. Oncol. (2009) 11, 80-91].

Even though IRN crosses the BBB, high intravenous doses between 125 and 500 mg/m² are required to achieve therapeutic levels in the brain resulting in serious systemic side effects including gastrointestinal toxicity, leading to early and late onset diarrhoea, and severe neutropenia. The issue of severe diarrhoea and neutropenia and the need to increase the levels of IRN in the brain has spurred on recent developments in improving IRN's ability to cross the BBB.

Local delivery of IRN directly to the brain tumour resection site could improve therapeutic outcomes by allowing for the delivery of larger doses directly to the tumour site, while reducing systemic concentrations and thus alleviating the aforementioned side-effects.

Baltes *et al* demonstrated a significant difference in the survival of GBM rats treated with 100-300 µm Drug Eluting Beads containing IRN, when compared to the placebo group [Baltes et al., J. Mater. Sci : Mater. Med. (2010), 21, 1393-1402]. Furthermore, there was no local toxicity associated or pronounced haemorrhaging around the area of implantation of the IRN beads. *In-vitro* IRN elution from the Drug Eluting Beads was fast.

A Phase I clinical trial demonstrated that IRN Drug Eluting Beads were safe for the local treatment of the tumour margin in GBM patients, with preliminary survival data as good as Gliadel^{®} when compared to historical controls [Cruickshank et al., Neuro. Oncol. (2015), 17, 11 [Clinical Trial Identifier: NCT02433392]]. However, the pharmacokinetic data demonstrated that the majority of IRN was removed from the brain within 48 hours, while all of it was removed by 72 hours.

EP1985286 A1 discloses water-insoluble, water-swellable, negatively-charged polymer microspheres for the delivery of positively-charged chemotherapeutic agents, including the preparation of IRN-loaded microspheres made from a 1:1 copolymer of PVA and 2-acrylamido-2-methylpropane sulphonate.

WO2014/059022 discloses polymer nanoparticles containing IRN and conjugated cisplatin, with the nanoparticles having an average diameter of about 55 nm.

There remains an unmet need to provide a drug delivery system for the treatment of brain tumours which is efficacious against even the most aggressive of tumours, whilst avoiding the unwanted side effects associated with current treatments.

The present invention was devised with the foregoing in mind.

### SUMMARY OF THE DISCLOSURE

In a first aspect, the present invention provides a chemotherapeutic seed comprising a biodegradable polymer and a drug selected from irinotecan, a pharmaceutically acceptable salt of irinotecan, 7-ethyl-10-hydroxy-camptothecin (SN-38)and a pharmaceutically acceptable salt of SN-38; wherein the seed has a primary length of at least 0.5 mm, and wherein primary length refers to the shortest dimension of the seed.

In another aspect, the present invention provides a chemotherapeutic seed consisting of a biodegradable polymer and a drug selected from IRN, a pharmaceutically acceptable salt of IRN, SN-38 and a pharmaceutically acceptable salt of SN-38; wherein the seed has a primary length of at least 0.5 mm.

In a further aspect, the present invention provides a seed as described herein for use in therapy.

In a further aspect, the present invention provides a seed as described herein for use in the treatment of a brain tumour, such as high grade gliomas (e.g. GBM).

In another aspect, the present invention provides a pharmaceutical composition comprising one or more seeds as described herein.

In a further aspect, the present invention provides a process for the preparation of a chemotherapeutic seed as described herein, the process comprising the steps of:
a) charging the drug and biodegradable polymer to a hot melt extruder;
b) extruding the mixture from the extruder;
c) forming the extrudate from step b) into one or more seeds of the required dimensions.

The above and further aspects of the invention are described in further detail herein.

### BRIEF DESCRIPTION OF THE DRAWINGS

Particular embodiments of the invention are further described hereinafter with reference to the accompanying drawings, in which:
**Figure 1** shows the influence of plasticizer type and loading on (A) the consistency of blank seed diameter during manufacture; and the swelling of the blank seeds in water when prepared using as plasticizer (B) Kolliphor^{®} RH40; (C) Kolliphor^{®} P188; and (D) Kolliphor^{®} P237.
**Figure 2** shows an image of 2mm diameter × 6mm length seeds with 30 wt% IRN HCL loading prepared according to Example 1.
**Figure 3** shows the extracted IRN HCl content of 2mm diameter × 3mm length seeds prepared with 10, 20, 30, 40 and 50 wt% IRN HCL loadings, as determined according to the method of Example 5.
**Figure 4** shows the extracted IRN HCl content of 30 wt% drug-loaded seeds with 2 × 2 mm, 2 × 3 mm and 2 × 6 mm dimensions, as determined according to the method of Example 5.
**Figure 5** shows the extracted IRN HCl content of 30 wt% drug-loaded 2 × 6 mm seeds, prepared according to Example 3 with (A) 10% plasticiser; 100 RPM screw speed; 150°C mixing temperature; **(B)** 0% plasticiser; 100 RPM screw speed; 150°C mixing temperature; (C) 0% plasticiser; 90 RPM screw speed; 150°C mixing temperature; **(D)** 0% plasticiser; 90 RPM screw speed; 140°C mixing temperature; and **(E)** 0% plasticiser; 90 RPM screw speed; 130°C mixing temperature.
**Figure 6** shows Raman images of cross-sections of 30 wt% IRN HCl-loaded 2x6 mm seeds, prepared according to Example 3 with **(A)** 10% plasticiser; 100 RPM screw speed; 150°C mixing temperature; **(B)** 0% plasticiser; 100 RPM screw speed; 150°C mixing temperature; (C) 0% plasticiser; 90 RPM screw speed; 150°C mixing temperature; **(D)** 0% plasticiser; 90 RPM screw speed; 140°C mixing temperature; and (E) 0% plasticiser; 90 RPM screw speed; 130°C mixing temperature. The IRN HCl is coloured dark for the mapping.
**Figure 7** shows a comparison of the cytotoxicity against primary GBM cells of a solution of IRN HCl which has been extracted from 2 × 3 mm seeds containing different loadings of IRN HCl, versus freshly prepared solutions of unprocessed IRN HCL of the same concentration - as determined according to Example 8.
**Figure 8** shows a comparison of the cytotoxicity against primary GBM cells of a solution of IRN HCl which has been extracted from 30 wt% seeds of different lengths, versus freshly prepared solutions of unprocessed IRN HCL of the same concentration - as determined according to Example 8.
**Figure 9** shows the cumulative *in vitro* elution of IRN HCl from 2 × 3 mm seeds containing different loadings of IRN HCl after 1-7 days incubation under (A) sink conditions; and (B) bio-relevant conditions - as described in Example 9.
**Figure 10** shows the cumulative *in vitro* elution of IRN HCl from 30 wt% seeds of different lengths after 1-7 days incubation under **(A)** sink conditions; and **(B)** bio-relevant conditions - as described in Example 9.
**Figure 11** shows the daily *in vitro* elution of IRN HCl for 7 days under bio-relevant conditions from **(A)** 2 × 3 mm seeds containing different loadings of IRN HCl; and **(B)** 30 wt% seeds of different lengths.
**Figure 12** shows the cumulative *in vitro* elution of IRN HCl from 30 wt% seeds prepared from different inherent viscosity PLGAs after 1-7 days incubation under sink conditions.
**Figure 13** shows the cytotoxicity against primary GBM cells of IRN HCl eluted at day 1 and day 7 from 2 × 3 mm seeds containing different drug loadings - as described in Example 10.
**Figure 14** shows the cytotoxicity against primary GBM cells of IRN HCl eluted at day 1 and day 7 from 30 wt% seeds of different lengths - as described in Example 10.
**Figure 15** shows the cytotoxicity against primary GBM cells of IRN HCl eluted at day 1 and day 7 from 30 wt% seeds prepared from different viscosity PLGAs - as described in Example 10.
**Figure 16** shows the cytotoxicity against primary GBM cells taken from the resection margin of GBM patient of 2 × 3 mm seeds containing different IRN HCl loadings at days 1, 2, 3, 4, 5 and 7 post-incubation - as described in Example 11.
**Figure 17** shows histological sections of mice brains after **(A)** just surgery (Sham); **(B)** treatment with drug-free placebo seeds and **(C), (D)** and **(E)** treatment with 30, 40 and 50 weight % respectively IRN HCl seeds - according to Example 12.
**Figure 18** shows the toxicity scores for mice brains treated with drug-free (0%) placebo seeds and 30, 40 and 50 weight % IRN HCl seeds over an 8-week period - according to Example 12.
**Figure 19** shows images of the mouse surgical site before incision, pre-resection (with the tumour identified), post resection and with the implant IRN HCl seeds in place - according to Example 13.
**Figure 20** shows Kaplan-Meier survival plots for resected mice with a U87-human glioblastoma cell line after: just surgery (Sham) treatment; treatment with drug-free (0%) placebo seeds; and treatment with 30, 40 and 50 wt% IRN HCl seeds - according to Example 13.
**Figure 21** shows images taken using a fluorescent microscope of a selection of the surviving mice at day 70 post-implantation.
**Figure 22** shows the *in vitro* cumulative percentage release **(A** and **B)** and daily release **(C** and **D)** of irinotecan from seeds containing 10%, 30% or 50% w/w IRN HCl loadings after 1-7 days incubation under sink conditions, where the seeds were manufactured using compression **(A** and **C)** or hot melt extrusion **(B** and **D).**
**Figure 23** shows the cell viability of primary GBM cells taken from 8 patients with recurrent GBM and after five days exposure to increasing concentrations of irinotecan (IRN), temozolomide (TMZ) or irinotecan (IRN) + pitavastatin (PVT).
**Figure 24** shows the cell viability of primary GBM cells taken from 4 patients with recurrent GBM and after three, five, seven, nine and 11 days exposure to 3.5 Log nM irinotecan, 5.0 Log nM temozolomide or 2.5 Log nM irinotecan + pitavastatin.
**Figure 25** shows: the *in vitro* **daily** release of drug from multi-layered seeds containing **(A)** 30% IRN and 50% PVT in individual layers and **(B)** 40% IRN and 50% PVT in invidual layers after 1-14 days incubation under sink conditions [the dashed lines are the amounts of IRN and PVT that need to be released in order to be effective based on the data from figures 23 and 24]; and the *in vitro* **cumulative** release of drug from multi-layered seeds containing **(C)** 30% IRN and 50% PVT in individual layers and **(D)** 40% IRN and 50% PVT in individual layers.
**Figure 26** shows the cytotoxicity of multi-layered seeds containing (i) no drug (control); (ii) 30% IRN and 50% PVT in individual layers; and (iii) 40% IRN and 50% PVT in individual layers; when the seeds were placed directly onto primary margin cells taken from a recurrent GBM patient.

### DETAILED DESCRIPTION

### Chemotherapeutic seeds

As described above, the present invention provides a chemotherapeutic seed comprising a biodegradable polymer and a drug selected from irinotecan, a pharmaceutically acceptable salt of irinotecan, 7-ethyl-10-hydroxy-camptothecin (SN-38) and a pharmaceutically acceptable salt of a derivative of SN-38; wherein the seed has a primary length of at least 0.5 mm, and wherein primary length refers to the shortest dimension of the seed.

By incorporating the drug into a biodegradable seed of the above dimensions it is possible to achieve sustained drug release from the seed over a period of at least 3 days under physiological conditions. This makes the seeds of the present invention suitable for efficacious local drug delivery to the resection site of brain tumours, especially GBM.

Irinotecan (IRN) has the following structure:

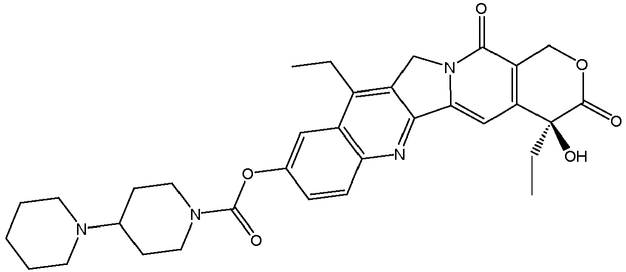

Derivatives of irinotecan include active metabolites of irinotecan, such as SN-38. SN-38 is the compound 7-ethyl-10-hydroxy-camptothecin, which has the following structure:

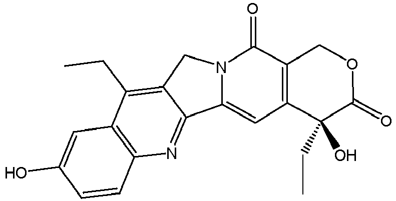

Pharmaceutically acceptable salts of a drug of the present invention include an acid-addition salt of a compound of the invention which is sufficiently basic, for example, an acid-addition salt with, for example, an inorganic or organic acid, for example hydrochloric, hydrobromic, sulfuric, phosphoric, trifluoroacetic, formic, citric or maleic acid. In addition a suitable pharmaceutically acceptable salt of a drug of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

In an embodiment, the drug is irinotecan. In an embodiment, the drug is a pharmaceutically acceptable salt of irinotecan, such as the hydrochloride salt. In an embodiment, the drug is irinotecan hydrochloride. It will be understood that the invention also comprises hydrates or solvates of the drugs of the invention. In a particular embodiment, the drug is a hydrate of irinotecan hydrochloride. In a further embodiment, the drug is irinotecan hydrochloride trihydrate.

Suitable biodegradable polymers are polymers capable of breaking down under physiological conditions such that the polymer and its degradation products do not invoke any unacceptable toxicity or immune response. Such polymers may be natural or synthetic in origin. Examples of natural biodegradable polymers include polysaccharides such as chitosan, alginate and dextran, or polyesters such as polyhydroxyalkanoates. Examples of synthetic biodegradable polymers include polymers of lactic acid and glycolic acid and copolymers thereof.

In an embodiment the biodegradable polymer is poly(lactic-co-glycolic acid) or polylactic acid, wherein each polymer is end-capped with either acid or ester groups. In a preferred embodiment, the biodegradable polymer is poly(lactic-co-glycolic acid). Poly(lactic-co-glycolic acid) is referred to herein also as PLGA. In an embodiment, the biodegradable polymer is PLGA end-capped with acid groups. In a preferred embodiment, the biodegradable polymer is PLGA end-capped with ester groups.

The acidity of the biodegradable polymer may be quantified by determining the acid number via titration. This technique is well-known in the art and typically involves dissolving a known amount of a polymer sample in an organic solvent (e.g. isopropanol) and titrating it with a solution of potassium hydroxide of known concentration using phenolphthalein as a colour indicator. The acid number is expressed as the mg of KOH required to neutralize 1 g of the sample. In an embodiment, the biodegradable polymer has an acid number less than or equal to 1 mg KOH/g. In an embodiment, the biodegradable polymer is PLGA and the PLGA has an acid number less than or equal to 1 mg KOH/g.

In an embodiment, the biodegradable polymer is PLGA, wherein the PLGA has a lactic acid weight content of about 5 to 95% with the balance being glycolic acid. In an embodiment, the PLGA has a lactic acid weight content of about 10 to 90%, such as about 20 to 80%, about 30 to 70%, or preferably about 40 to 60%; with the balance being glycolic acid. In an embodiment, the PLGA has a lactic acid:glycolic acid weight ratio of about 5:95, about 15:85, about 25:75, about 40:60, about 50:50, about 60:40, about 75:25, about 85:15 or about 95:5. In a preferred embodiment, the PLGA has a lactic acid:glycolic acid weight ratio of about 40:60, about 50:50 or about 60:40. In a most preferred embodiment, the PLGA has a lactic acid:glycolic acid weight ratio of about 50:50. In a most preferred embodiment, the PLGA is end-capped with ester groups and has a lactic acid:glycolic acid weight ratio of about 50:50. In a most preferred embodiment, the PLGA has a lactic acid:glycolic acid weight ratio of about 50:50 and an acid number less than or equal to 1 mg KOH/g.

In an embodiment, the biodegradable polymer is PLGA, wherein the PLGA has a number average molecular weight of about 2 to 15 kDa, such as about 5 to 15 kDa, or about 5 to 12 kDa.

In an embodiment, the PLGA has a lactic acid:glycolic acid weight ratio about 50:50 and a number average molecular weight of about 2 to 15 kDa. In an embodiment, the PLGA is end-capped with ester groups, has a lactic acid:glycolic acid weight ratio about 50:50 and a number average molecular weight of about 2 to 15 kDa. In a preferred embodiment, the PLGA has a lactic acid:glycolic acid weight ratio of about 50:50, an acid number less than or equal to 1 mg KOH/g and a number average molecular weight of about 2 to 15 kDa.

In an embodiment, the PLGA has an inherent viscosity of 0.15 to 1.2 dL/g, when measured at 25 °C in chloroform at a concentration of 0.5 g/dL. In a preferred embodiment, the PLGA has an inherent viscosity of 0.2 to 1.2 dL/g, such as 0.25 to 1.2 dL/g, 0.3 to 1.0 dL/g, 0.3 to 0.8 dL/g, or 0.3 to 0.6 dL/g when measured at 25 °C in chloroform at a concentration of 0.5 g/dL. In a most preferred embodiment, the PLGA has an inherent viscosity of 0.3 to 0.5 dL/g when measured at 25 °C in chloroform at a concentration of 0.5 g/dL.

In an embodiment, the PLGA has a lactic acid:glycolic acid weight ratio about 50:50, a number average molecular weight of about 2 to 15 kDa and an inherent viscosity of 0.3 to 0.5 dL/g, when measured at 25 °C in chloroform at a concentration of 0.5 g/dL. In an embodiment, the PLGA has a lactic acid:glycolic acid weight ratio about 50:50 and an inherent viscosity of 0.3 to 0.5 dL/g, when measured at 25 °C in chloroform at a concentration of 0.5 g/dL. In an embodiment, the PLGA has a lactic acid:glycolic acid weight ratio about 50:50, an acid number less than or equal to 1 mg KOH/g and an inherent viscosity of 0.3 to 0.5 dL/g, when measured at 25 °C in chloroform at a concentration of 0.5 g/dL. In an embodiment, the PLGA has a lactic acid:glycolic acid weight ratio about 50:50, an acid number less than or equal to 1 mg KOH/g, a number average molecular weight of about 2 to 15 kDa and an inherent viscosity of 0.3 to 0.5 dL/g, when measured at 25 °C in chloroform at a concentration of 0.5 g/dL.

The loading of drug within the chemotherapeutic seed can be chosen dependent on the required levels of active agent at the local delivery site and the period of drug cover required post-implantation. In an embodiment, the drug is present in a loading of 1 to 50 weight % (wt%) relative to the weight of the seed. In an embodiment, the drug is present in a loading of 5 to 45 wt %, such as 5 to 45 wt %, 10 to 40 wt %, 30 to 45 wt %, 25 to 45 wt %, or 30 to 40 wt % relative to the weight of the seed. In an embodiment, the drug is present in a loading of 5 to 50 wt %, such as 10 to 50 wt %, 20 to 50 wt %, 25 to 50 wt %, or 30 to 50 wt % relative to the weight of the seed. In an embodiment, the drug is present in a loading of about 30 wt % or about 40 wt % relative to the weight of the seed.

In an embodiment, the drug is irinotecan, or a pharmaceutically acceptable salt of irinotecan and the drug is present in a loading of 25 to 45 wt %, or 30 to 40 wt % relative to the weight of the seed. In an embodiment, the drug is irinotecan, or a pharmaceutically acceptable salt of irinotecan and the drug is present in a loading of about 30 wt %, or about 40 wt % relative to the weight of the seed.

In an embodiment, the drug is a pharmaceutically acceptable salt of irinotecan (preferably irinotecan hydrochloride) and the drug is present in a loading of 25 to 45 wt %, or 30 to 40 wt % relative to the weight of the seed. In an embodiment, the drug is a pharmaceutically acceptable salt of irinotecan (preferably irinotecan hydrochloride) and the drug is present in a loading of about 30 wt %, or about 40 wt % relative to the weight of the seed.

In an embodiment, the drug is SN-38, or a pharmaceutically acceptable salt of SN-38and the drug is present in a loading of 1 to 20 wt %, 1 to 10 wt %, or 1 to 5 wt % relative to the weight of the seed.

A chemotherapeutic seed according the present invention can take various shapes, such as sphere, plate, rod, cylinder, cuboid or cube. The method of preparation of the seed may influence the shape of the seed produced. For example, the shape and size of seeds prepared by extrusion can be controlled by the choice of extrusion die. Preferably the seeds of the present invention are rods. As the skilled person will understand, a rod has a shape which is cylindrical. In a preferred embodiment, the seed is substantially cylindrically-shaped.

A seed according to the present invention has a primary length of at least 0.5 mm. As used herein, the term 'primary length' refers to the shortest dimension of the seed. In a spherical seed this would be the diameter of the sphere and in a cube this would be the length of the cube. In shapes, such as a cuboid or a cylinder, which have dimensions of different lengths, the primary length is the shortest length, while the secondary length is the second shortest length (see schematic representation below). 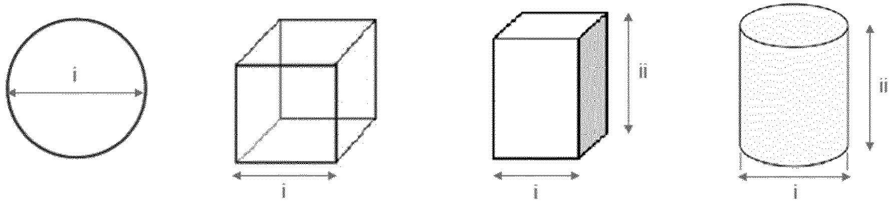 Schematic representation of possible seed shapes (I-r: sphere, cube, cuboid and cylinder) with the primary length (i) of each shape shown, as well as the secondary length (ii) for the cuboid and cylinder.

Accordingly, when the seed is substantially cylindrically-shaped, the primary length is the diameter of the cylinder and the secondary length is the length of the cylinder. In an embodiment, the seed is substantially cylindrically-shaped, the primary length is the seed diameter and the secondary length is the seed length.

It is understood that when the seed comprises one or more pores or cavities, then the primary length is defined in terms of the dimensions of the whole seed.

The size of the seed is important to deliver the desired drug release profile. In an embodiment, the seed has a primary length of at least 1 mm, such as at least 1.5 mm. In an embodiment, the seed has a primary length of 0.5 to 5 mm, such as 0.5 to 4 mm, 1 to 4 mm, or preferably 1 to 3 mm. Seeds having primary lengths within the range of about 0.5 to 5 mm, are particularly suited to administration to the tumour site via a catheter. In a preferred embodiment, the seed has a primary length of about 2 mm.

In an embodiment, the seed has a secondary length of at least 1 mm, such as at least 1.5 mm. In an embodiment, the seed has a secondary length of 1 to 10 mm, such as 1 to 8 mm, 1 to 7 mm, or preferably 2 to 6 mm. In a preferred embodiment, the seed has a secondary length of about 2 mm, about 3 mm, about 4 mm, about 5 mm or about 6mm.

In a preferred embodiment, the seed has a primary length of about 2 mm and a secondary length of about 3 mm or about 6 mm. In a preferred embodiment, the seed is substantially cylindrically-shaped and has a primary length of about 2 mm and a secondary length of about 3 mm or about 6 mm.

Seeds prepared according to the present invention may be prepared with or without plasticisers. In an embodiment, the seed does not contain a plasticizer. In order to be suitable for local implantation (e.g. in the brain parenchyma) in terms of minimizing the risk of adverse reactions, it is desirable that the seeds are non-swelling and that they have a smooth surface (characterized by an absence of so-called 'shark skinning'). It may therefore be necessary for the seeds to contain one or more plasticisers. In an embodiment, the seed further comprises a pharmaceutically acceptable plasticizer. Suitable plasticisers include poloxamers (such as Kolliphor^{®} P188, Kolliphor^{®} P237 and Kolliphor^{®} P407), polyethylene glycols (such as Kollisolv^{®} PEG 300 and Kollisolv^{®} PEG 400), polyvinyl acetate, stearic acid, glyceryl behenate, Triacetin, diethyl phthalate, glyceryl monostearate, triethyl citrate and macrogolglycerol hydroxystearate (such as Kolliphor^{®} RH 40). In an embodiment, the plasticiser is selected from poloxamers, polyethylene glycols, polyvinyl acetate and macrogolglycerol hydroxystearate. In a preferred embodiment, the plasticiser is chosen from Kolliphor^{®} P188, Kolliphor^{®} P237 and Kolliphor^{®} RH 40. In a more preferred embodiment, the plasticiser is a poloxamer, such as Kolliphor^{®} P 188 or Kolliphor^{®} P 237. Most preferably, the plasticiser is Kolliphor^{®} P 188.

Experiments using Kolliphor^{®} P188, Kolliphor^{®} P237 or Kolliphor^{®} RH 40 plasticisers to prepare blank (i.e. drug-free) PLGA seeds via hot melt extrusion showed that low loadings (5 % w/w) resulted in seeds with inconsistent average diameters, whilst high loadings (15-20 % w/w) typically resulted in the seeds showing swelling when placed in water for up to 48 hours - see Comparative Example 1. Therefore, optimal plasticizer loading is in the range 5-15 % w/w. In an embodiment, the seed comprises plasticizer at a loading of about 5-15 weight %, preferably about 10 weight %, relative to the weight of the seed. In a preferred embodiment, the seed comprises Kolliphor^{®} P 188 at a loading of about 10 weight % relative to the weight of the seed.

In an embodiment, the seeds according to the present invention only contain biodegradable polymer and drug and optionally plasticizer. Therefore, in an aspect of the invention there is provided a chemotherapeutic seed consisting of a biodegradable polymer and a drug selected from irinotecan, a pharmaceutically acceptable salt of irinotecan, a derivative of irinotecan and a pharmaceutically acceptable salt of a derivative of irinotecan; wherein the seed has a primary length of at least 0.5 mm. In an embodiment, there is provided a chemotherapeutic seed consisting of a biodegradable polymer (preferably PLGA) and a pharmaceutically acceptable salt of irinotecan (preferably irinotecan hydrochloride); wherein the seed has a primary length of at least 0.5 mm. In a preferred embodiment, there is provided a chemotherapeutic seed consisting of a biodegradable polymer (preferably PLGA) and a pharmaceutically acceptable salt of irinotecan (preferably irinotecan hydrochloride); wherein the seed has a primary length of 1 to 4 mm. In a further aspect of the invention there is provided a chemotherapeutic seed consisting of a biodegradable polymer, a plasticiser and a drug selected from irinotecan, a pharmaceutically acceptable salt of irinotecan, a derivative of irinotecan and a pharmaceutically acceptable salt of a derivative of irinotecan; wherein the seed has a primary length of at least 0.5 mm. In an embodiment, there is provided a chemotherapeutic seed consisting of a biodegradable polymer (preferably PLGA), a plasticizer (preferably Kolliphor^{®} P 188) and a pharmaceutically acceptable salt of irinotecan (preferably irinotecan hydrochloride); wherein the seed has a primary length of at least 0.5 mm. In a preferred embodiment, there is provided a chemotherapeutic seed consisting of a biodegradable polymer (preferably PLGA), a plasticizer (preferably Kolliphor^{®} P 188) and a pharmaceutically acceptable salt of irinotecan (preferably irinotecan hydrochloride); wherein the seed has a primary length of 1 to 4 mm.

### Drug Release Profiles

Irinotecan works during the S-Phase of the cell cycle, which occurs around day 3, which means the efficacy of irinotecan could be improved if therapeutic levels could be maintained at the tumour site for at least 5 to 7 days.

As described above, a device for local delivery of a chemotherapeutic agent, such as irinotecan, directly to a solid tumour resection site could improve therapeutic outcomes by allowing for the delivery of larger doses of the drug directly in the first 24-48 hours, followed by a prolonged period of slower drug release for at least 5 to 7 days, post device implantation. Such a release profile (`initial burst' followed by prolonged release) is particularly suited to the efficacious delivery of irinotecan to solid tumour resection sites, as (i) the initial burst allows for a high local dose of drug to be delivered initially, enhancing diffusion of the drug through the surrounding tissue and killing off most of the residual tumour cells not removed surgically, while (ii) the sustained period of release provides a 'top-up' of drug to kill those cells that were in the G₁ or G₂ phase of the cell cycle and did not enter the S-Phase of the cell cycle until around day 3 post implantation.

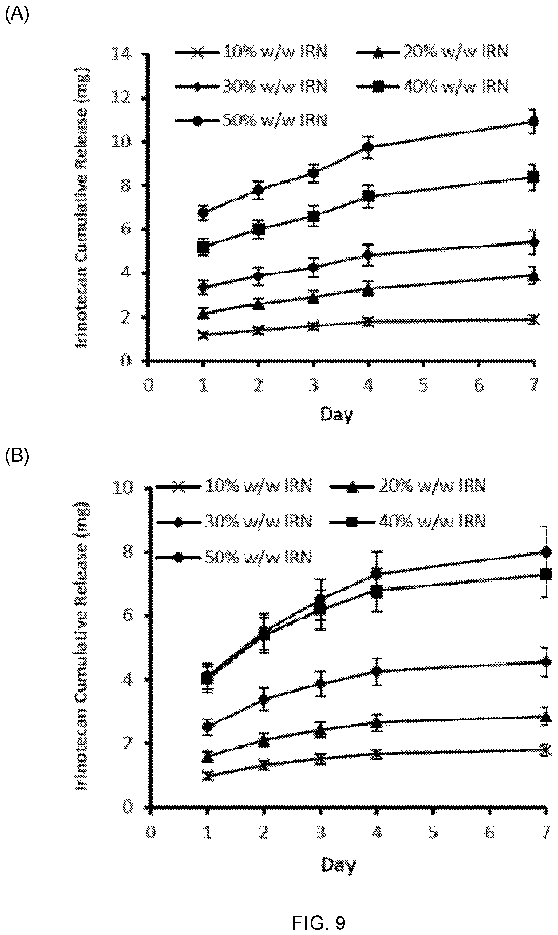

Chemotherapeutic seeds of the present invention are capable of providing a drug release profile as described above. Experiments conducted measuring the release of drug from the seeds into aqueous sink and bio-relevant media over a period of 7 days, show an initial burst of drug release by day 1, followed by a steady slow release of drug out to 7 days post-incubation (Example 9 and Figs. 9-12).

Suitably, the 'initial burst' release phase is defined as the seed releasing between 40% and 80% of the drug after 1 day post-incubation in aqueous media, relative to the total amount of drug released. More suitably, the 'initial burst' is defined as the seed releasing between 50% and 75% of the drug after 1 day post-incubation in aqueous media, relative to the total amount of drug released.

Suitably, the 'prolonged release' phase is defined as the seed releasing between 20% and 60% of the drug between day 1 and day 7 post-incubation in aqueous media, relative to the total amount of drug released. More suitably, the 'prolonged release' is defined as the seed releasing between 25% and 50% of the drug between day 1 and day 7 post-incubation in aqueous media, relative to the total amount of drug released.

An example of a suitable *in vitro* test to measure the drug release profile of seeds according to the present invention are described below in Example 9. In summary, individual seeds (n = 4) were placed into a sealed flask containing either 3 mL of water (bio-relevant medium designed to mimic cerebral spinal fluid) or 5 mL of phosphate buffered (pH 7.4) saline solution (sink conditioned media) and placed into an orbital shaking incubator at 37°C and 60 rpm. Complete media replacement was performed at 1, 2, 3, 4, and 7 days. The samples where filtered using a 0.45µm filter and analysed for IRN content using HPLC.

An aqueous physiological-type environment is defined as being either bio-relevant or sink-conditioned water-based media, as described hereinabove.

In an embodiment of the present invention, there is provided a seed as described herein, wherein the seed continuously releases the drug over a period of at least 3 days, at least 4 days, at least 5 days, at least 6 days or at least 7 days when placed in an aqueous physiological-type environment.

In an embodiment, the seed, when placed in an aqueous physiological-type environment, releases:
i. more than 20% drug after 1 day;
ii. more than 30% drug after 3 days;
iii. more than 40% drug after 5 days; and/or
iv. more than 50% drug after 7 days;
relative to the total amount of drug released.

In an embodiment, the seed, when placed in an aqueous physiological-type environment, releases:
i. more than 40% drug after 1 day;
ii. more than 50% drug after 3 days;
iii. more than 60% drug after 5 days; and/or
iv. more than 70% drug after 7 days;
relative to the total amount of drug released.

In an embodiment, the seed, when placed in an aqueous physiological-type environment, releases:
i. more than 50% drug after 1 day;
ii. more than 60% drug after 3 days;
iii. more than 70% drug after 5 days; and/or
iv. more than 80% drug after 7 days;
relative to the total amount of drug released.

In an embodiment, the seed, when placed in an aqueous physiological-type environment, releases:
i. Up to 80% drug after 1 day; and/or
ii. Up to 95% drug after 3 days;
relative to the total amount of drug released after 7 days.

In an embodiment, the seed, when placed in an aqueous physiological-type environment, releases:
i. Up to 75% drug after 1 day; and/or
ii. Up to 95% drug after 3 days;
relative to the total amount of drug released after 7 days.

In an embodiment, the seed, when placed in an aqueous physiological-type environment, releases:
i. Up to 65% drug after 1 day; and/or
ii. Up to 85% drug after 3 days;
relative to the total amount of drug released after 7 days.

In an embodiment, the seed, when placed in an aqueous physiological-type environment, releases:
i. 40 to 80% drug after 1 day;
ii. 50 to 95% drug after 3 days; and/or
iii. 60 to 100% drug after 5 days
relative to the total amount of drug released after 7 days.

In an embodiment, the seed, when placed in an aqueous physiological-type environment, releases:
i. 50 to 75% drug after 1 day;
ii. 60 to 95% drug after 3 days; and/or
iii. 70 to 100% drug after 5 days
relative to the total amount of drug released after 7 days.

In an embodiment, the seed, when placed in an aqueous physiological-type environment, releases:
i. 60 to 70% drug after 1 day;
ii. 70 to 90% drug after 3 days; and/or
iii. 80 to 100% drug after 5 days
relative to the total amount of drug released after 7 days.

In an embodiment, when the seed is placed an aqueous physiological-type environment, the cumulative drug release after 1 day is 1 to 6 mg and after 3 days is 1 to 7.5 mg. In an embodiment, when the seed is placed an aqueous physiological-type environment, the cumulative drug release after 1 day is 1 to 6 mg; after 3 days is 1 to 7.5 mg; and after 7 days is 2 to 10 mg.

In an embodiment, the seed, when placed in an aqueous physiological-type environment, releases at least 300 to 1000 µg of drug per day for a period of at least 5 days, at least 6 days or at least 7 days. In a further embodiment, the seed, when placed in an aqueous physiological-type environment, releases at least 300 to 1000 µg of irinotecan (or a pharmaceutically acceptable salt thereof) per day for a period of at least 5 days, at least 6 days or at least 7 days. In a yet further embodiment, the seed, when placed in an aqueous physiological-type environment, releases 2000 to 5000 µg of irinotecan (or a pharmaceutically acceptable salt thereof) after 1 day, followed by at least 300 µg of irinotecan (or a pharmaceutically acceptable salt thereof) per day for the subsequent 6 days.

In an embodiment, the seed comprises 30-40% w/w of irinotecan hydrochloride and when placed in an aqueous physiological-type environment, the seed releases:
i. 40 to 70% irinotecan after 1 day;
ii. 60 to 90% irinotecan after 3 days; and/or
iii. 70 to 100% irinotecan after 5 days
relative to the total amount of irinotecan released after 7 days.

In an embodiment, the seed has a primary length of about 2 mm, comprises 30-40% w/w of irinotecan hydrochloride and when placed in an aqueous physiological-type environment, the seed releases:
i. 40 to 70% irinotecan after 1 day;
ii. 60 to 90% irinotecan after 3 days; and/or
iii. 70 to 100% irinotecan after 5 days
relative to the total amount of irinotecan released after 7 days.

In an embodiment, the seed has a primary length of about 2 mm, a secondary length of about 3 to 6 mm, comprises 30-40% w/w of irinotecan hydrochloride and when placed in an aqueous physiological-type environment, the seed releases:
i. 40 to 70% irinotecan after 1 day;
ii. 60 to 90% irinotecan after 3 days; and/or
iii. 70 to 100% irinotecan after 5 days
relative to the total amount of irinotecan released after 7 days.

In any of the above embodiments, placing of a seed in an aqueous physiological-type environment may refer to incubation of a seed in 5 mL of phosphate buffered (pH 7.4) saline solution at 37°C with agitation at 60 rpm and complete media replacement being carried out at 1, 2, 3, 4, and 7 days post-incubation. In any of the above embodiments, placing of a seed in an aqueous physiological-type environment may refer to incubation of a seed in 3 mL of water at 37°C with agitation at 60 rpm and complete media replacement being carried out at 1, 2, 3, 4, and 7 days post-incubation.

In an embodiment, the seed, when incubated in 5 mL of pH 7.4 phosphate buffered saline solution at 37°C with agitation at 60 rpm and complete media replacement being carried out at 1, 2, 3, 4, and 7 days post-incubation, releases:
i. 50 to 75% drug after 1 day;
ii. 60 to 95% drug after 3 days; and/or
iii. 70 to 100% drug after 5 days
relative to the total amount of drug released after 7 days.

In an embodiment, the seed, when incubated in 5 mL of pH 7.4 phosphate buffered saline solution at 37°C with agitation at 60 rpm and complete media replacement being carried out at 1, 2, 3, 4, and 7 days post-incubation, releases at least 300 to 1000 µg of drug per day for a period of at least 5 days, at least 6 days or at least 7 days.

In any of the above embodiments describing the drug release profile, the term 'drug' may refer to irinotecan (or a pharmaceutically acceptable salt thereof), or 7-ethyl-10-hydroxy-camptothecin (SN-38) (or a pharmaceutically acceptable salt thereof). Preferably, 'drug' refers to irinotecan (or a pharmaceutically acceptable salt thereof).

### Compositions

In an aspect of the present invention there is provided a pharmaceutical composition comprising one or more seeds as described herein. In an embodiment, the pharmaceutical composition comprises 10-100 seeds as described herein. In a preferred embodiment, the pharmaceutical composition comprises 30-60 seeds as described herein.

In an embodiment, the pharmaceutical composition comprises one or more seeds, wherein each seed has a primary length of at least 0.5 mm and comprises a biodegradable polymer in which is dispersed a drug selected from irinotecan, a pharmaceutically acceptable salt of irinotecan, 7-ethyl-10-hydroxy-camptothecin (SN-38) and a pharmaceutically acceptable salt of SN-38.

In an embodiment, the pharmaceutical composition comprises one or more seeds, wherein each seed has a primary length of about 2 mm and comprises PLGA in which is dispersed irinotecan hydrochloride.

In an embodiment, the pharmaceutical composition comprises one or more seeds, wherein each seed has a primary length of about 2 mm, a secondary length of about 3 to 6 mm and comprises PLGA in which is dispersed irinotecan hydrochloride.

In an embodiment, the pharmaceutical composition comprises one or more seeds, wherein each seed has a primary length of about 2 mm, a secondary length of about 3 to 6 mm and comprises PLGA (such as 50:50 w/w LA:GA) in which is dispersed 30-40% w/w irinotecan hydrochloride.

The compositions of the present invention may comprise one or more additional therapeutic agents. The additional therapeutic agents may have an anti-cancer effect or any other therapeutic effect, such as being an antibiotic, anti-inflammatory, anticoagulant, analgesic, statin, antiplatelet, antifungal, angiotensin-converting enzyme (ACE) inhibitor, enzyme acetaldehyde dehydrogenase inhibitor or other suitable, complementary therapeutic agent. In an embodiment, the composition further comprises an additional anti-cancer agent. In an embodiment, the one or more additional therapeutic agents are selected from anti-cancer agents (such as bevacizumab), statins (such a pitavastatin), antiplatelet agents (such as ticlopidine), antifungal agents (such as itraconazole), angiotensin-converting enzyme (ACE) inhibitors (such as captopril), enzyme acetaldehyde dehydrogenase inhibitors (such as disulfiram) and anti-inflammatory agents (such as celecoxib).

In an embodiment, there is provided a pharmaceutical composition comprising one or more seeds as described herein and a statin (such as pitavastatin). In an embodiment, there is provided a pharmaceutical composition comprising one or more seeds as described herein and an angiotensin-converting enzyme (ACE) inhibitor (such as captopril). In an embodiment, there is provided a pharmaceutical composition comprising one or more seeds as described herein and an enzyme acetaldehyde dehydrogenase inhibitor (such as disulfiram). In an embodiment, there is provided a pharmaceutical composition comprising one or more seeds as described herein, a statin (such as pitavastatin) and an angiotensin-converting enzyme (ACE) inhibitor (such as captopril). In an embodiment, there is provided a pharmaceutical composition comprising one or more seeds as described herein, a statin (such as pitavastatin) and an enzyme acetaldehyde dehydrogenase inhibitor (such as disulfiram). In an embodiment, there is provided a pharmaceutical composition comprising one or more seeds as described herein, an angiotensin-converting enzyme (ACE) inhibitor (such as captopril) and an enzyme acetaldehyde dehydrogenase inhibitor (such as disulfiram).

In an embodiment, the additional anti-cancer agent comprises one or more radioactive isotopes suitable for the treatment of cancer. Alternatively, the additional therapeutic agent is a radiosensitising agent for concurrent or subsequent radiotherapy treatment.

The additional therapeutic agent may be present in the composition in an admixture with the seeds. Alternatively, in an embodiment, the seeds may comprise two layers, wherein a first layer contains a drug selected from irinotecan, a pharmaceutically acceptable salt of irinotecan, a derivative of irinotecan and a pharmaceutically acceptable salt of a derivative of irinotecan; and a second layer contains an additional therapeutic agent. The seeds may further comprise additional layers and/or further therapeutic agents. In an embodiment, all the layers are made of biodegradable polymer, such as PLGA. In a further embodiment, the first layer is made of one biodegradable polymer, such as PLGA, and the second layer is made of a different biodegradable polymer. In an embodiment, there is provided a seed according to the present invention, the seed further comprising one or more additional therapeutic agents selected from anti-cancer agents (such as bevacizumab), statins (such as pitavastatin), antiplatelet agents (such as ticlopidine), antifungal agents (such as itraconazole), angiotensin-converting enzyme (ACE) inhibitors (such as captopril), enzyme acetaldehyde dehydrogenase inhibitors (such as disulfiram), anti-inflammatory agents (such as celecoxib) and copper gluconate. In an embodiment, there is provided a seed according to the present invention, the seed further comprising pitavastatin. In an embodiment, there is provided a seed according to the present invention, the seed further comprising captopril and disulfiram. In an embodiment, there is provided a seed according to the present invention, the seed further comprising captopril, celecoxib and itraconazole. In an embodiment, there is provided a seed according to the present invention, the seed further comprising captopril, pitavastatin and ticlopidine. In an embodiment, there is provided a seed according to the present invention, the seed further comprising pitavastatin, disulfiram and copper gluconate.

Pitavastatin refers to the compound ((3R,5S,6E)-7-[2-cyclopropyl-4-(4-fluorophenyl)quinolin-3-yl]-3,5-dihydroxyhept-6-enoic acid) having the following structure and pharmaceutically acceptable salts thereof:

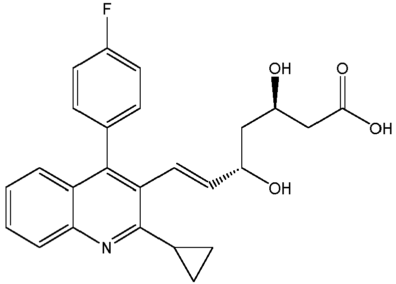

Conveniently, as used herein, pitavastatin refers to the calcium salt of the above compound, i.e. calcium bis((3R,5S,6E)-7-[2-cyclopropyl-4-(4-fluorophenyl)quinolin-3-yl]-3,5-dihydroxyhept-6-enoate).

In another embodiment, the composition comprises one or more seeds, wherein the seeds comprises a single layer of biodegradable polymer in which are dispersed:
a) a drug selected from irinotecan, a pharmaceutically acceptable salt of irinotecan, a derivative of irinotecan and a pharmaceutically acceptable salt of a derivative of irinotecan; and
b) an additional therapeutic agent (such as one of the therapeutic agents described above).

In one embodiment, the composition comprises one or more seeds, wherein each seed comprises two layers of biodegradable polymer in which are dispersed:
a) in a first layer a drug selected from irinotecan, a pharmaceutically acceptable salt of irinotecan, a derivative of irinotecan and a pharmaceutically acceptable salt of a derivative of irinotecan; and
b) in a second layer an additional therapeutic agent (such as one of the therapeutic agents described above).

In one embodiment, the composition comprises one or more seeds, wherein each seed comprises two layers of biodegradable polymer in which are dispersed:
a) in a first layer a drug selected from irinotecan, a pharmaceutically acceptable salt of irinotecan, a derivative of irinotecan and a pharmaceutically acceptable salt of a derivative of irinotecan; and
b) in a second layer pitavastatin.

In one embodiment, the composition comprises one or more seeds, wherein each seed comprises two layers of biodegradable polymer in which are dispersed:
a) in a first layer irinotecan hydrochloride; and
b) in a second layer pitavastatin.

In one embodiment, the composition comprises one or more seeds, wherein each seed comprises two layers of PLGA (such as 50:50 w/w LA:GA) in which are dispersed:
a) in a first layer irinotecan hydrochloride; and
b) in a second layer pitavastatin.

In one embodiment, the composition comprises one or more seeds, wherein each seed comprises two layers of PLGA (such as 50:50 w/w LA:GA) in which are dispersed:
a) in a first layer 30-40% w/w irinotecan hydrochloride; and
b) in a second layer 10-50% (such as 20-40%) w/w pitavastatin.

In one embodiment, the composition comprises one or more seeds, wherein each seed has a primary length of about 2 mm and a secondary length of about 6 mm and comprises two layers of PLGA (such as 50:50 w/w LA:GA) in which are dispersed:
a) in a first layer 30-40% w/w irinotecan hydrochloride; and
b) in a second layer 10-50% (such as 20-40%) w/w pitavastatin.

In one embodiment, the composition comprises one or more seeds, wherein each seed has a primary length of about 2 mm and a secondary length of about 6 mm and comprises two layers of PLGA (such as 50:50 w/w LA:GA) in which are dispersed:
a) in a first layer 30-40% w/w irinotecan hydrochloride; and
b) in a second layer 10-50% (such as 20-40%) w/w pitavastatin;
wherein each of the two layers has a primary length of about 2 mm and a secondary length of about 3 mm.

### Therapeutic Uses

In an aspect of the present invention there is provided a seed as described herein for use in therapy. In an embodiment, there is provided a seed as described herein for use in the treatment of a disease or condition treatable by a drug selected from irinotecan, a pharmaceutically acceptable salt of irinotecan, 7-ethyl-10-hydroxy-camptothecin (SN-38)and a pharmaceutically acceptable salt of SN-38.

There is also provided a method for administering a drug selected from irinotecan, a pharmaceutically acceptable salt of irinotecan, 7-ethyl-10-hydroxy-camptothecin (SN-38) and a pharmaceutically acceptable salt of 7-ethyl-10-hydroxy-camptothecin (SN-38)to a warm-blooded animal (preferably a human), said method comprising implanting one or more seeds as described herein in the warm blooded animal.

In a further aspect of the present invention there is provided a seed as described herein for use in treating cancer. In a further aspect of the present invention there is provided a seed as described herein for use in treating solid tumours. In an embodiment, the cancer is a solid tumour selected from colorectal cancer, prostate cancer, pancreatic cancer, breast cancer, liver cancer, ovarian cancer, bladder cancer, lung cancer (including small cell lung cancer) or brain cancer. In a preferred embodiment, there is provided a seed as described herein for use in the treatment of a brain tumour; preferably wherein the brain tumour is a high grade (grades III and IV) glioma. In an embodiment, the seed is for use in the treatment of a grade IV glioma (such as glioblastoma multiforme).

The chemotherapeutic seeds of the present invention, or compositions comprising them, may enhance the effects of conventional chemotherapy, radiotherapy, immunotherapy, hormone therapy or gene therapy.

The chemotherapeutic seeds of the present invention, or compositions comprising them, may be placed directly in, or injected around, a solid tumour. Preferably, they may be placed into the resection cavity created after surgical debulking of a solid tumour. In an embodiment, there is provided a seed as described herein for use in the treatment of a solid tumour, wherein the treatment comprises implanting the seed (or seeds) into the resection margin of the debulked tumour. Alternatively, they may be inserted intra-tumourally in unresectable tumours by stereotaxy. In a preferred embodiment, there is provided a seed as described herein for use in the treatment of a brain tumour (preferably high grade gliomas (grades III and IV)), wherein the treatment comprises implanting the seed into the resection margin of the debulked brain tumour.

There is also provided a method for treating a brain tumour (preferably high grade gliomas (grades III and IV)), wherein the treatment comprises implanting a seed as described herein in or around the brain tumour (preferably wherein the treatment comprises implanting the seed into the resection margin of the debulked brain tumour).

Due to the size of the seeds as described herein, it is envisaged that the seeds may be administered to the tumour site via a catheter or needle. In an embodiment, a seed according to the present invention, or a composition comprising one or more of said seeds, is to be administered via a catheter or needle.

### Preparation of the chemotherapeutic seeds

The chemotherapeutic seeds as described herein may be prepared by any suitable method, including compression, solvent casting, hot moulding, injection moulding, extrusion, or 3D printing. The nature of the biodegradable polymer and/or the drug may influence the choice of manufacturing method. Preferably, the chemotherapeutic seeds as described herein are prepared by hot melt extrusion.

According to a further aspect of the invention there is provided a process for the preparation of a chemotherapeutic seed as described herein, the process comprising the steps of:
a) charging the drug and biodegradable polymer to a hot melt extruder;
b) extruding the mixture from the extruder;
c) forming the extrudate from step b) into one or more seeds of the required dimensions.

Preferably, the drug and biodegradable polymer are pre-mixed before being charged to the hot melt extruder. Therefore, in an embodiment, step a) comprises the steps of:
a1) mixing the drug and biodegradable polymer; and
a2) charging the mixture from step a1) to a hot melt extruder.

The mixing in step a1) may be carried out by any conventional means, such as by using a pharmaceutical mixer or a speed mixer.

If a plasticiser is a component of the seed being produced, then the plasticiser is also charged during step a) of the above process.

Preferably, the hot melt extruder is a co-rotating twin screw hot melt extruder. In an embodiment, the screw speed is 20 to 200 RPM, such as 50 to 150 RPM or preferably 80 to 120 RPM.

Typically, the hot melt extruder comprises three zones; firstly a feeding zone, then a mixing zone and finally a metering or discharge zone. To enable efficient melting of the biodegradable polymer and homogenous mixing with the drug particles, but without causing thermal degradation, typically the three zones will be run at different temperatures. In an embodiment, the feeding zone is set at 25 to 175 °C. In an embodiment, the mixing zone is set at 100 to 200 °C. In an embodiment, the discharge zone is set at 50 to 150 °C. In an embodiment, the feeding zone is set at 25 to 175 °C, the mixing zone is set at 100 to 200 °C and the discharge zone is set at 50 to 150 °C.

In step b) the mixture is typically extruded from the discharge zone of the hot melt extruder via a die on to a haul off conveyor. After cooling on the haul off conveyor, the extrudate solidifies. Once solidified the extrudate may be formed into one or more seeds of the required dimensions (step c)). Typically, the extrudate is cut into seeds of the desired length (such as 1 to 10 mm, preferably 2 to 6 mm). The diameter of the seeds is determined by the diameter of the die aperture and the haul off conveyor speed. Preferably the diameter of the seeds is 0.5 to 5 mm, such as 1 to 3 mm, or most preferably about 2 mm. In a preferred embodiment, the diameter of the die aperture is 0.5 to 10 mm, such as 2 to 5 mm. In a preferred embodiment, the haul off conveyor speed is 50 to 250 RPM, such as 100 to 200 RPM. Preferably, diameter of the die aperture is 0.5 to 10 mm (such as 2 to 5 mm) and the haul off conveyor speed is 50 to 250 RPM (such as 100 to 200 RPM).

In an embodiment, the biodegradable polymer in step a) is PLGA, preferably the PLGA has a lactic acid:glycolic acid weight ratio about 50:50. In an embodiment, the drug in step a) is irinotecan hydrochloride. In a preferred embodiment, the biodegradable polymer in step a) is PLGA (preferably the PLGA has a lactic acid:glycolic acid weight ratio about 50:50) and the drug in step a) is irinotecan hydrochloride. In a preferred embodiment, the biodegradable polymer in step a) is PLGA wherein the PLGA is ester end-capped and has a lactic acid:glycolic acid weight ratio about 50:50; and the drug in step a) is irinotecan hydrochloride. In a preferred embodiment, the biodegradable polymer in step a) is PLGA with an acid number less than or equal to 1 mg KOH/g and a lactic acid:glycolic acid weight ratio about 50:50; and the drug in step a) is irinotecan hydrochloride.

In a preferred embodiment, there is provided a process for the preparation of a chemotherapeutic seed as described herein comprising PLGA and a drug selected from irinotecan, a pharmaceutically acceptable salt of irinotecan, 7-ethyl-10-hydroxy-camptothecin (SN-38) and a pharmaceutically acceptable salt of SN-38, the process comprising the steps of:
a) charging the drug (preferably irinotecan hydrochloride) and PLGA (preferably 50:50 w/w LA:GA) to a hot melt extruder;
b) extruding the mixture from the extruder at a screw speed of 90-110 RPM; and
c) forming the extrudate from step b) into one or more seeds of the required dimensions;
wherein the hot melt extruder has a feeding zone set at 50 to 150 °C (such as 150 °C), a mixing zone set at 125 to 155 °C (such as 150 °C) and a discharge zone set at 70 to 110 °C (such as 100 °C). In a further embodiment, wherein the seed further comprises a plasticiser, the screw speed is about 100 RPM and the mixing zone is set at about 150 °C. In a further alternative embodiment, wherein the seed comprises no plasticiser, the screw speed is about 90 RPM and the mixing zone is set at about 130 °C.

In a further embodiment there is provided a process for the preparation of a chemotherapeutic seed as described herein comprising PLGA, irinotecan hydrochloride and an additional therapeutic agent, the process comprising the steps of:
a) charging irinotecan hydrochloride, the additional therapeutic agent and PLGA to a hot melt extruder;
b) extruding the mixture from the extruder;
c) forming the extrudate from step b) into one or more seeds of the required dimensions.

In a further embodiment there is provided a process for the preparation of a chemotherapeutic seed as described herein comprising PLGA, irinotecan hydrochloride and an additional therapeutic agent, wherein the seed comprises two layers of which a first layer contains irinotecan hydrochloride; and a second layer contains an additional therapeutic agent; the process comprising the steps of:
a) charging irinotecan hydrochloride and PLGA to a hot melt extruder;
b) extruding the mixture from the extruder;
c) forming the extrudate from step b) into one or more seeds of the required dimensions;
d) charging the additional therapeutic agent and PLGA to a hot melt extruder;
e) extruding the mixture from the extruder;
f) forming the extrudate from step e) into one or more seeds of the required dimensions;
g) physically joining the seeds from step c) to the seeds from step f) to form seeds comprising two layers.

In the above embodiment, preferably in step c) and/or step f) the extrudate is cut into seeds of the desired length (such as 1 to 10 mm, preferably about 3 mm). In the above embodiment, preferably in step c) and/or step f) the extrudate is formed into seeds of diameter about 2 mm. In the above embodiment, preferably in step c) and/or step f) the extrudate is formed into seeds of diameter about 2 mm and length about 3 mm. In the above embodiment, preferably in step d) the additional therapeutic agent is pivatastatin. In the above embodiment, preferably in step g) the physical joining of the seeds is achieved by butt welding.

In an aspect of the present invention, there is provided a chemotherapeutic seed obtained, or obtainable, by a process as described herein. In an embodiment, there is provided a chemotherapeutic seed obtained, or obtainable, by a hot melt extrusion process as described herein.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

### EXAMPLES

The invention will now be described in more detail in relation to the following illustrative examples.

### Abbreviations

- DCM: dichloromethane
- DMEM: Dulbecco's Modified Eagle Medium
- GBM: glioblastoma multiforme
- HME: hot melt extrusion
- HPLC: high performance liquid chromatography
- IRN: irinotecan
- IRN HCl: irinotecan hydrochloride
- NIH: National Institutes of Health
- PBS: phosphate buffered saline
- PLGA: poly(lactide-co-glycolide)
- PSI: pounds per square inch
- PVT: pitavastatin
- RPM: revolutions per minute
- TMZ: temozolomide

### Materials & Apparatus

Poly(DL-lactide-co-glycolide) (PLGA) with a 50:50 lactide:glycolide ratio (PURASORB PDLG)) was purchased from Corbion (Amsterdam, The Netherlands). Samples '5002', '5004' and '5010' were described as having inherent viscosities of 0.16-0.24 dL/g, 0.32-0.48 dL/g and 0.8-1.2 dL/g respectively when measured at 25 °C in chloroform at a concentration of 0.5 g/dL. The '5002' PLGA was acid end-capped (acid number ≥ 6 mg KOH/g) , while the '5004' and `5010' PLGA samples were ester end-capped (acid number ≤ 1 mg KOH/g). Irinotecan hydrochloride (IRN HCl) was purchased from LGM Pharma (Nashville, TN). Pitavastatin (calcium salt) was purchased from LGM Pharma (Nashville, TN). Temozolomide, Kolliphor^{®} P188, acetonitrile, dichloromethane and sodium phosphate were purchased from Sigma-Aldrich (Dorset, England). Kolliphor^{®} plasticisers RH40 and P237 were purchased from BASF (Ludwigshafen, Germany). GBM cell lines were retrieved from patients who received resection surgery at the Queen Elizabeth Hospital, Birmingham, UK.

Hot melt extrusion was carried out using a 10 mm 40:1 microlab co-rotating twin screw extruder (Rondol Technology, Stoke-on-Trent, UK).

Confocal Raman spectroscopy was used to detect and spatially resolve IRN distribution within the extruded PLGA polymer blocks. Maps and spectra were acquired using a confocal Raman microscope (Alpha300R, WITec, Ulm, Germany), equipped with an Acton SP2300 Imaging Monochromator/Spectrograph (Princeton Instruments, MA, USA), fitted with a 300g/mm 750nm blaze grating, and a 785nm 250mW diode laser (XTRA II, Toptica photonics, Munich, Germany). Raman maps were acquired a 20X (NA = 0.45) objective lens and in continuous scan mode with an integration time of 1s and scan areas of 5mm wide by 2mm high. Data were accumulated and exported using the WITec Control software version 1.6 (WITec, Ulm, Germany) into .spc files, which were then processed using in-house MATALB Raman data processing tools. Imported hyperspectral datasets were baseline corrected before applying vector normalisation and finally generation of IRN and PLGA maps.

### General Method for the preparation of IRN HCl-loaded biodegradable polymer seeds

The IRN HCl-loaded biodegradable polymer seeds are manufactured by mixing IRN HCL with a biodegradable polymer using a pharmaceutical mixer or speed mixer. Depending on the weight % of IRN HCL within the implant, addition of a plasticiser may be required. The mix is then fed into the barrel of the hot melt extruder. The biodegradable polymer (and plasticiser if required) soften and/or melt and the IRN HCL or derivatives thereof are incorporated into the biodegradable polymer matrix. The mix is discharged from the hot melt extruder via a die, whereby the haul off conveyor speed is used to control the diameter of the extrudate strand. Upon cooling, the extrudate is cut into one or more seeds of the required dimensions.

### Comparative Example 1: Manufacture of blank PLGA seeds to assess the influence of plasticiser on seed diameter consistency and swelling resistance

The appropriate amount of PLGA polymer and plasticizer (5, 10, 15 and 20% w/w) - of either Kolliphor^{®} P188, Kolliphor^{®} P237 or Kolliphor^{®} RH40 - were weighed into a sealed plastic container and roll mixed for 10 minutes. The active mix was subsequently fed into a 40:1 microlab extruder at a feed rate of 90 grams per hour. The feeding, mixing and metering zones of the extruder were set at 45°C, 110°C and 70°C respectively. The melt was extruded through a 2mm die and subsequently cut into blank PLGA seeds of 6mm in length.

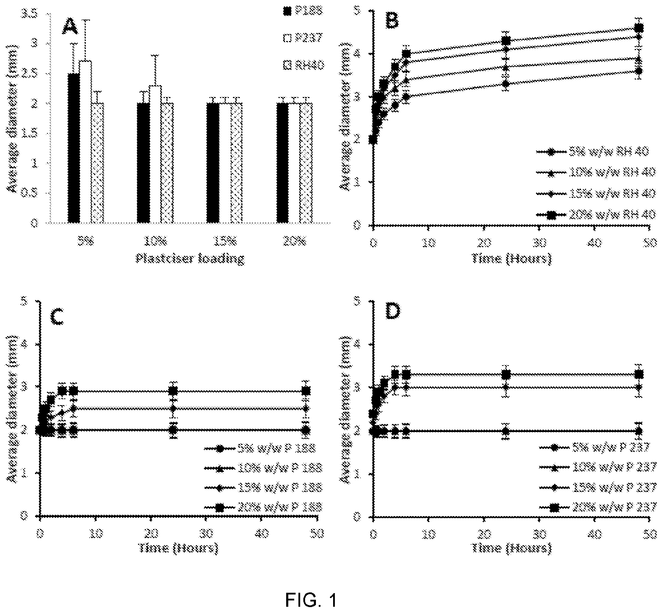

To determine the diameters and weights of the blank seeds, samples (n = 10) of each blank seed were assessed for their average weight and diameter. Each seed was measured in the middle and at both ends, using a digital Vernier calliper. The three measurements were averaged to give the diameter of each individual blank seed. The results can be seen in Figure 1A. The higher the plasticiser loading the more consistent the seed diameter produced, with Kolliphor^{®} P188 and RH 40 requiring a minimum loading of 10% w/w to produce seeds with a consistent diameter, while Kolliphor^{®} P237 required a minimum loading of 15% w/w.

To investigate the influence of the plasticiser type and loading on the swelling of the blank seeds, each blank seed (n = 4) was placed in a glass vial with 3ml of distilled water and the vials placed into an orbital shaking incubator (Infors HT) at 37°C and 60 RPM. Their length and width were measured using a digital Vernier calliper at 0, 0.5, 1, 2, 4, 6, 24 and 48 hours post-incubation. The RH 40 plasticizer resulted in significant (P = 0.025) swelling at all loadings (Figure 1B). The P188 and P237 plasticizers had significant (P = 0.019) swelling at loadings of 15 and 20% w/w (Figs. 1C and 1D). Based on the data shown in Figure 1, the preferred plasticiser in terms of consistent seed diameter and resistance to swelling was P188 at a loading of 10% w/w.

### Example 1: Preparation of IRN HCl-loaded PLGA seeds

10 wt% seeds were prepared by mixing 1g of IRN HCl with 9g of '5004' PLGA. 20 wt% seeds were prepared by mixing 2g of IRN HCl with 8g of '5004' PLGA. 30 wt% seeds were prepared by mixing 3g of IRN HCl with 7g of '5004' PLGA.

Each of the mixes was individually fed into the barrel of the hot melt extruder. For all mixes the feed rate was set at 4.5g per minute and the screw speed of the extruder was set at 90 RPM. The barrel of the hot melt extruder had three heating zones with heating zone 1 (feeding zone) at 45°C and heating zone 2 (mixing zone) set at 130°C, while heating zone 3 (discharge zone) was set at 100 °C. The torque of the extruder was between 4 and 8 N m, dependent on the composition of the mix being fed into the extruder. The die on the extruder was 4 mm and the haul off conveyor had a speed of 150 RPM to maintain the diameter of the extrudate at 2 mm. Once solidified the extrudate was cut into seeds of 3 mm in length. The extrudate containing 30 wt% of IRN HCl was also cut into seeds of either 2 mm or 6 mm in length.

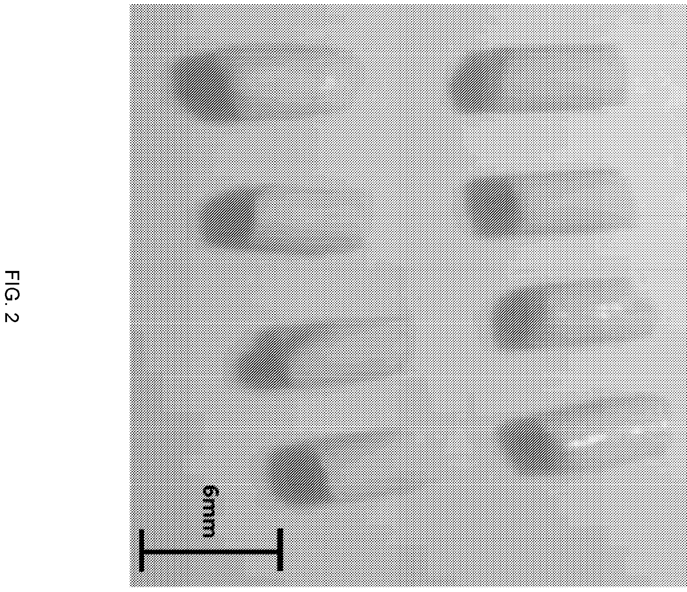

Figure 2 shows the appearance of 30 wt% IRN HCl seeds with 2 mm diameter and 6 mm length.

### Example 2: Preparation of IRN HCl-loaded PLGA seeds containing 10 wt% plasticiser

30 wt% seeds were prepared by mixing 3g of IRN HCl with 6g of '5004' PLGA and 1g of Kolliphor^{®} P188 plasticiser. 40 wt% seeds were prepared by mixing 4g of IRN HCl with 5g of '5004' PLGA and 1g of Kolliphor^{®} P188 plasticiser. 50 wt% seeds were prepared by mixing 5g of IRN HCl with 4g of '5004' PLGA and 1g of Kolliphor^{®} P188 plasticiser.

Each of the mixes was individually fed into the barrel of the hot melt extruder. For all mixes the feed rate was set at 4.5g per minute and the screw speed of the extruder was set at 100 RPM. The barrel of the hot melt extruder had three heating zones with heating zone 1 (feeding zone) was at 45 °C and heating zone 2 (mixing zone) set at 100 °C, while heating zone 3 (discharge zone) was set at 70 °C. The torque of the extruder was between 4 and 8 N m, and depended on the composition of the mix being fed into the extruder. The die on the extruder was 4 mm and the haul off conveyor had a speed of 150 RPM to maintain the diameter of the extrudate at 2 mm. Once solidified the extrudate was cut into seeds of 2 mm or 3 mm in length.

### Example 3: Alternative preparation of 30 wt% IRN HCl-loaded PLGA seeds without plasticiser

30 wt% IRN HCl-loaded PLGA seeds having 2 mm diameter and 6 mm length were prepared according to Example 2, under the following hot melt extrusion parameters:
(A) 10% plasticiser; 100 RPM screw speed; 150 °C mixing temperature (comparative sample containing plasticiser)
(B) 0% plasticiser; 100 RPM screw speed; 150 °C mixing temperature;
(C) 0% plasticiser; 90 RPM screw speed; 150 °C mixing temperature;
(D) 0% plasticiser; 90 RPM screw speed; 140 °C mixing temperature; and
(E) 0% plasticiser; 90 RPM screw speed; 130 °C mixing temperature;

### Example 4: Preparation of 30 wt% IRN HCl-loaded PLGA seeds with different viscosity PLGA

30 wt% IRN HCl-loaded PLGA seeds having 2 mm diameter and 6 mm length were prepared according to Example 2, substituting either '5002' or `5010' PLGA for '5004' PLGA.

### Example 5: Content uniformity and drug stability of the IRN HCl-loaded PLGA seeds

A random sample (n = 10) of each seed type was selected, weighed and placed into a glass vial. DCM (10 mL) was added to each vial and left for 1 hour to dissolve the seeds. Once dissolved, the vials were placed in a water bath set at 40 °C to completely evaporate the dichloromethane. The remaining residue was re-suspended in HPLC mobile phase, causing the PLGA polymer to crash out and the IRN HCl to go into solution. To ensure complete dissolution of IRN HCl the solution was sonicated for 30 minutes. The solution was then filtered and analysed for its IRN HCl concentration using the HPLC methodology described below.

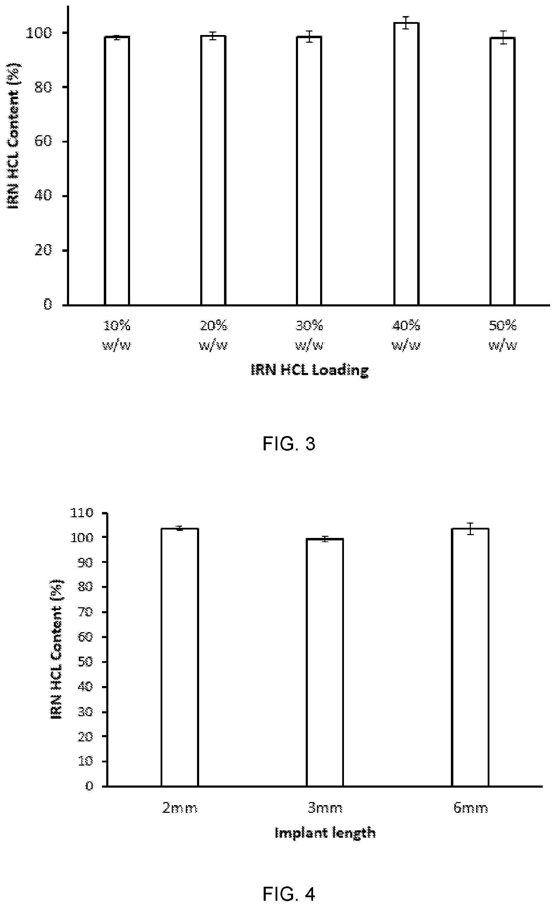

Figures 3 and 4 show that the seeds tested had drug contents between 98% and 104% of their theoretical value, confirming that the hot melt extrusion process produced seeds with the desired drug content.

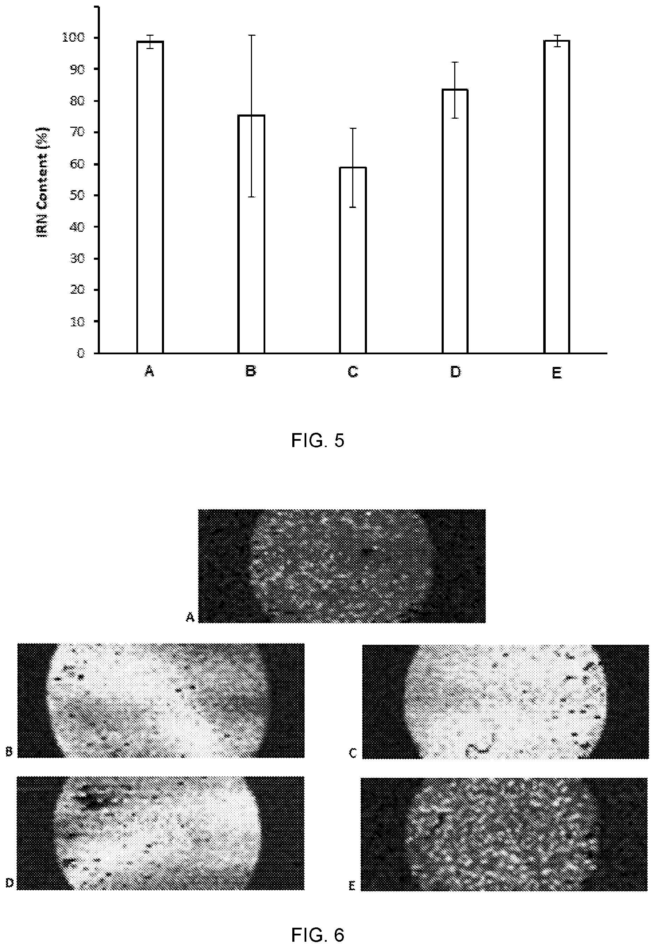

Figure 5 shows the drug content variation for the 30% w/w seeds prepared according to Example 3 (A) to (E). At 100 RPM and 150°C mixing, 10% w/w of a plasticiser is required to ensure the homogenous mixing of the IRN into the PLGA. As it may be desirable to produce seeds without the need for a plasticizer, alternative hot melt extrusion parameters were explored as detailed in Example 3. Upon removing the plasticiser the average content of the IRN in the seeds significantly reduced, while the homogeneity was poor with a standard deviation of 25.6 (Fig. 5B). The screw speed was reduced to 90 RPM to increase the residence time and improve mixing, however the average drug content was further reduced (Fig. 5C) due to degradation of the IRN. By reducing the mixing temperature to 130 °C and maintaining the screw speed at 90 RPM it was possible to achieve an acceptable IRN content and homogeneity (Fig. 5E - std dev of 1.8) similar to that of the seeds with 10% plasticizer.

Raman spectroscopic mapping carried out on cross-sections of the Example 3 seeds (Fig. 6) confirmed that in Example 3 (A) and Example 3 (E) the drug is evenly distributed across the seeds. In Examples 3 (B), (C) and (D) the drug is not evenly distributed across the seed and the overall intensity of the dark spots is less, indicating lower drug content.
**IRN HPLC methodology** - HPLC analysis was performed on a Dionex Ultimate 3000 HPLC with a Phenomenex Luna C18 4.6 × 150 mm column having a 5µM particle size. The mobile phase was comprised of 75% pH 2.7 phosphate buffer and 25% acetonitrile. The flow rate was 1.00mL/min, while UV detection was performed at a wavelength of 225nm with an injection volume of 20µL. Linearity was found to be in the range of 0.01 to 10mg/ml with an R² of 1.00.

### Example 6A: Preparation of IRN-HCl (30% w/w) and PVT (50% w/w) loaded multi-layer seeds

3g of IRN HCl was mixed with 7g of '5004' PLGA to form mix A. 5g of PVT was separately mixed with 5g of '5004' PLGA to form mix B.

Mix A was fed into the barrel of a hot melt extruder. The feed rate was set at 4.5g per minute and the screw speed of the extruder was set at 90 RPM. The barrel of the hot melt extruder had three heating zones with heating zone 1 (feeding zone) was at 45 °C and heating zone 2 (mixing zone) set at 130°C, while heating zone 3 (discharge zone) was set at 70 °C. The torque of the extruder was between 4 and 8 N m, and depended on the composition of the mix being fed into the extruder. The die on the extruder was 4 mm and the haul off conveyor had a speed of 150 RPM to maintain the diameter of the extrudate at 2 mm. Once solidified the extrudate was cut into (A) seeds of 3 mm in length. The above hot melt extrusion process was repeated with mix B to give (B) seeds of 3 mm in length.

Dual layer implants were prepared by butt welding (A) seeds to (B) seeds as follows: a pallet knife was heated to 120 °C and an (A) seed and a (B) seed pushed against opposite sides of the knife. The knife was removed and the melted ends of each layer were pushed together and held for 60 seconds to allow the polymer to cool and the layers to weld together. The dimensions of the dual-layer seeds were 2 mm diameter × 6 mm length.

### Example 6B: Preparation of IRN-HCl (40% w/w) and PVT (50% w/w) loaded multi-layer seeds

4g of IRN HCl was mixed with 6g of '5004' PLGA to form mix A. 5g of PVT was separately mixed with 5g of '5004' PLGA to form mix B. Dual layer implants were prepared from these mixes in an analogous manner to the method described for Example 6A.

### Example 7: Preparation of 10%, 30% or 50% w/w IRN-HCl loaded seeds by (A) hot melt extrusion and (B) compression

10 wt% mix was prepared by mixing 1g of IRN HCl with 9g of '5004' PLGA. 30 wt% mix was prepared by mixing 3g of IRN HCl with 7g of '5004' PLGA. 50 wt% mix was prepared by mixing 5g of IRN HCl with 5g of '5004' PLGA.

### A) Hot melt extrusion

Each of the 3 mixes was individually fed into the barrel of the hot melt extruder. For all mixes the feed rate was set at 4.5g per minute and the screw speed of the extruder was set at 90 RPM. The barrel of the hot melt extruder had three heating zones with heating zone 1 (feeding zone) was at 45 °C and heating zone 2 (mixing zone) set at 130 °C while heating zone 3 (discharge zone) was set at 70 °C. The torque of the extruder was between 4 and 8 N m, and depended on the composition of the mix being fed into the extruder. The die on the extruder was 4 mm and the haul off conveyor had a speed of 150 RPM to maintain the diameter of the extrudate at 2 mm. Once solidified the extrudate was cut into seeds of 6 mm in length.

### B) Compression

Each of the 3 mixes was individually fed into a 2 mm diameter pellet die and subsequently placed into a 15 Ton hydraulic KBr press. The mixes were compressed under 7.5 tonnes (116.67 PSI) of pressure for 5 minutes at room temperature. The compressed implants were cut into seeds of 6 mm in length.

### Example 8: Determination of the cytotoxicity of irinotecan within the PLGA seeds on GBM cells

400µL of the IRN content solutions from Example 5 were diluted with 3600µL of sterile cell culture media and filtered. Control solutions were produced by dissolving the required amount (based on the actual content of IRN in each seed) of IRN in 3mL of DCM and subsequently evaporating it off. The residual IRN was dissolved in 3mL of PBS and 400µL of this solution was diluted with 3600µL of cell culture media and filtered. The cytotoxicity of the solutions was determined against primary GBM cell lines and using a 5 day exposure time.

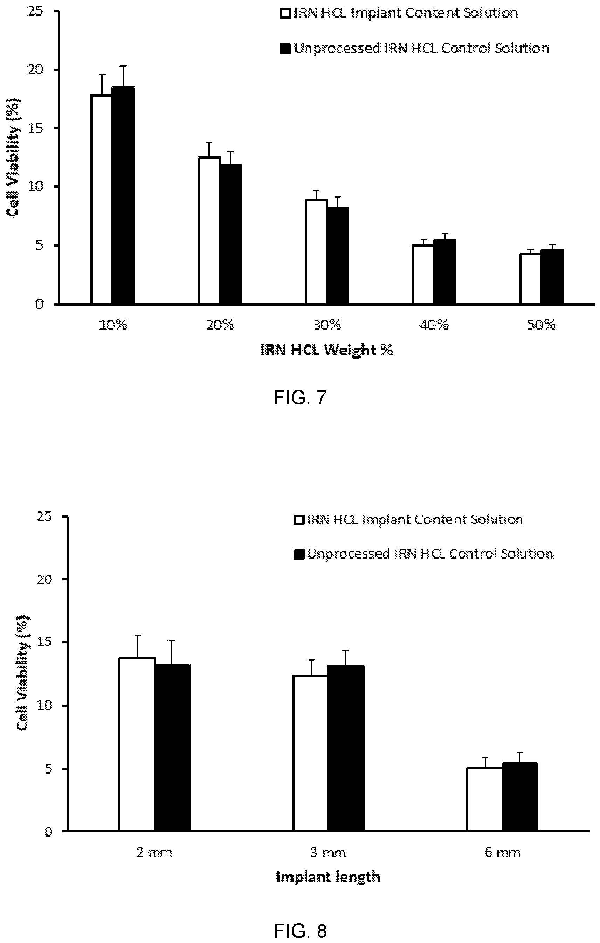

Figures 7 and 8 show the GBM cell viability data generated according to Example 8 and demonstrate that the IRN extracted from the various seeds retained its cytotoxicity. When compared to the unprocessed control solutions of the same concentrations, there was no significant (P = 0.562) difference in cell viability across the samples.

### Example 9: In vitro release of IRN from the PLGA seeds into both sink conditioned and bio-relevant release media

Individual seeds of 2 × 3 mm dimensions with varying (10, 20, 30, 40 and 50% w/w) IRN HCl loadings and individual seeds of 30% w/w IRN HCl loading with varying (2, 3 and 6 mm) lengths (n = 4) were placed into a sealed flask containing either 3 mL of water (bio-relevant media) or 5 mL of phosphate buffered (pH 7.4) solution (sink conditioned media) and placed into an orbital shaking incubator (Unitron HT infors) at 37 °C and 60 rpm. Complete media replacement was performed at 1, 2, 3, 4, and 7 days. The samples where filtered using a 0.45µm filter and analysed for IRN content using the IRN HPLC method described above.

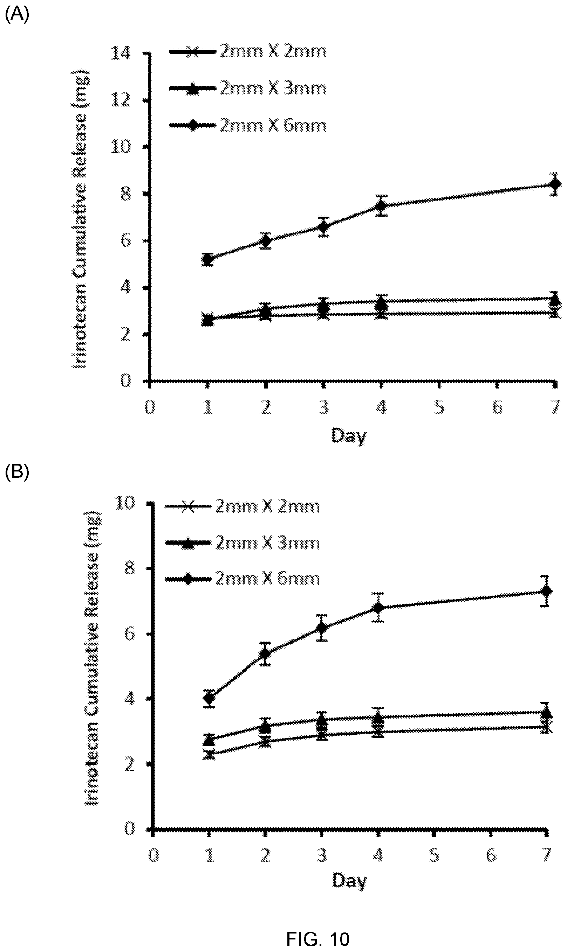

Figures 9 and 10 show the measured *in vitro* release profiles under both sink (A) and bio-relevant (B) conditions. All sample demonstrated an 'initial burst' release over the first 24 hours, followed by a prolonged period of slower release until day 7. The burst release was proportionate to both the loading of IRN HCl and the seed length. Under sink conditions, an increase in drug loading from 10 to 50% significantly (P = 0.0103) increased release on day one from 1.2 mg to 6.8 mg, while the total release over the 7 days increased (P = 0.0111) from 1.9 mg to 10.9 mg (Figure 9A). Under bio-relevant conditions, however, there was no significant (P = 0.582) difference in release between the 40 and 50% w/w seeds. This is potentially due to the large amount of IRN on the surface and within the seeds being released and saturating the diffusion layer at the seed/release media interface. An increase in drug loading from 10 to 40/50% significantly (P = 0.0213) increased release on day one from 1.0 mg to approximately 4.0 mg, while the total release over the 7 days increased (P = 0.0237) from 1.8 mg to approximately 8 mg (Figure 9B).

Increasing the dimensions of the seeds from 2 × 2 mm to 2 × 3 mm had no significant influence on drug release (Figure 10). However, increasing the dimensions to 2 × 6 mm significantly increased release, with an increase on day one from 2.6 mg to 5.2 mg and an increase in the total release from 2.9 mg to 8.4 mg under sink conditions (Fig. 10A) and an increase on day one from 2.3 mg to 4.0 mg and an increase in the total release from 3.2 mg to 7.3 mg under bio-relevant conditions (Fig. 10B).

These results demonstrated that IRN release can be controlled by both drug loading and the seed dimensions, which facilitates accurate control of IRN levels *in vivo.* Under sink conditions all of the seed formulations released greater than 92% of their drug-loading over 7 days of release. Under the bio-relevant release conditions the seed formulations released between 71 and 102% of their drug-loading over 7 days of release.

The IRN levels released in the bio-relevant media were lower than those released under sink conditions. It is possible that the increased drug release under sink conditions was due to the increased volume (5 mL compared to 3 mL) of the media rather than its composition. It is postulated that the increased volume decreases the thickness and concentration of the IRN diffusion layer at the seed/release media interface, which increases drug release.

It is believed that this type of release profile (an initial burst in the first 24 hours, followed by a sustained period of release over 7 days) is clinically advantageous in the treatment of resected brain tumours, as the burst allows for a high local dose of IRN HCl to be delivered initially, enhancing diffusion of the IRN HCl through the brain tissue and killing off most of the residual tumour cells, while the sustained period of release provides a 'top-up' of IRN HCl to kill those tumour cells that were in the G₁ or G₂ phase of the cell cycle.

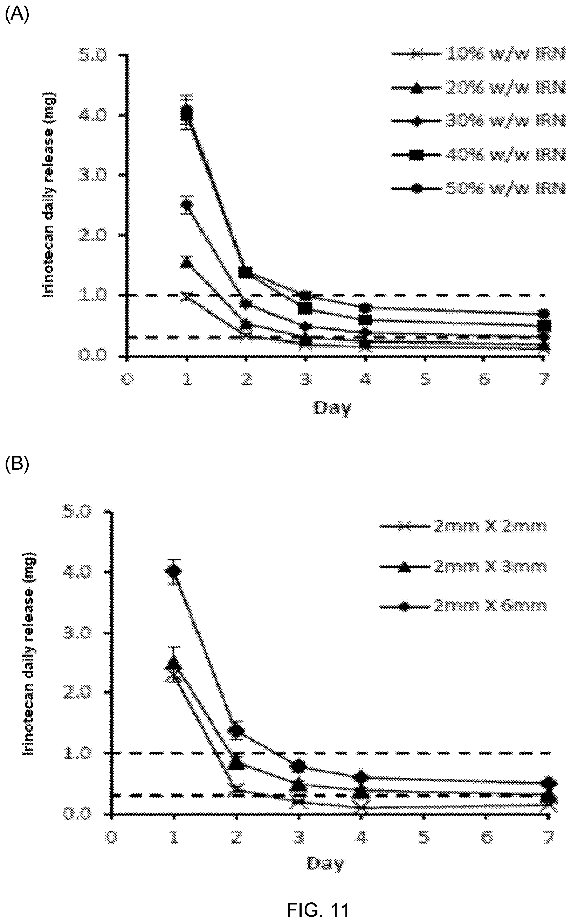

The *in vitro* daily release profiles for the seeds released under bio-relevant conditions are presented in Figure 11A and B, and also illustrate this drug release profile. There was a large day one burst, which increased significantly (P = 0.0213) with an increase in drug loading. The seeds then demonstrated a typical matrix release profile, with a decrease in drug release over time. The 2 × 3 mm 30%, 40% and 50% w/w and the 2 × 6 mm 30% w/w seed formulations were capable of releasing at least 300 to 1000 µg of IRN per day for the full 7 days (as represented by the lower and upper dotted lines in Figure 11A and B). Accordingly, in terms of the drug release profile the preferred drug loading is 30% to 50% w/w and the preferred seed size is 2 × 3 mm or 2 × 6 mm.

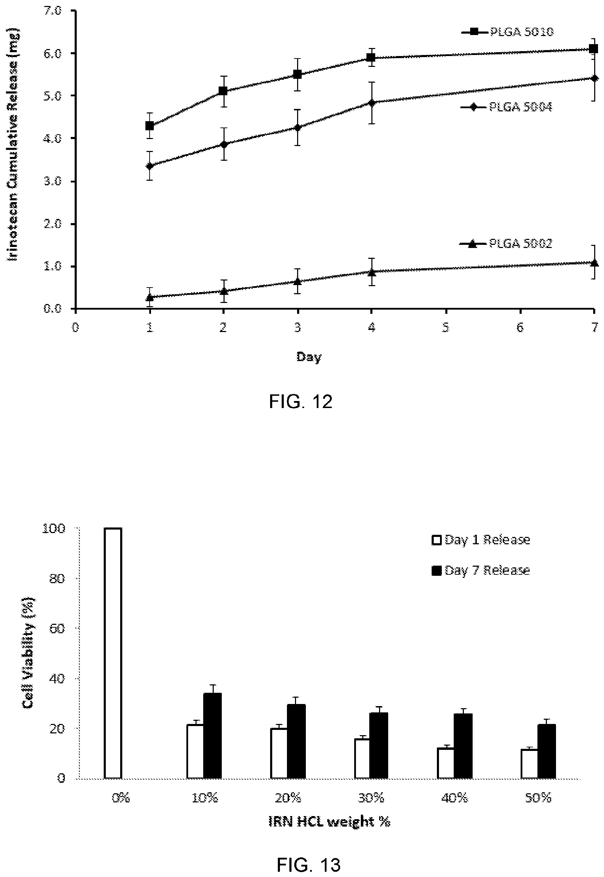

The 30% w/w IRN HCl PLGA seeds prepared according to Example 4 were also assessed for their *in vitro* drug release profile under sink conditions (Figure 12). Although the same overall profiles (initial burst followed by sustained release over 7 days) were seen for the 3 different PLGA viscosity formulations, the '5002' seeds surprisingly released significantly less drug than the '5004' or '5010' seeds. It is postulated that this is a result of the '5002' PLGA being acid-terminated, whereas the '5004' and `5010' PLGA samples were ester-terminated. The parent drug in the salt IRN HCl is positively charged and so it is possible that interactions between the polymer acid groups and the drug result in a stabilisation effect, such that release of the drug into the aqueous dissolution media is retarded.

### Example 10: Determination of the GBM cell cytotoxicity of the Irinotecan released under bio-relevant condition from the PLGA seeds

A 1 mL sample of the bio-relevant release media from day 1 and day 7 of the Example 9 drug release experiments was evaluated for cytotoxicity against primary GBM cells. 400µl of each bio-relevant release sample was added to 3600µl of cell culture media and filtered using a syringe filter to ensure sterility. 200µl was subsequently added to the wells of a 96-well plate containing the cultured primary GBM cells and 200 µl of cell culture media. Following 5 days of exposure an MTT assay was performed to assess % cell viability.

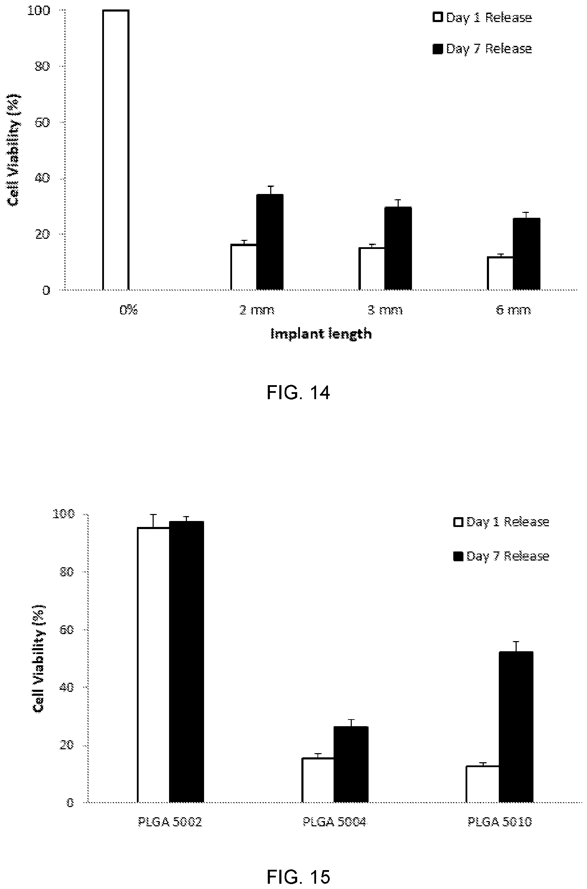

An increase in IRN loading from 10 to 50% w/w resulted in an a decrease in the cell viability from 21.3 to 11.2% (Figure 13 - day 1 release), while an increase in the length of the seeds from 2 to 6 mm resulted in a decrease in cell viability from 34.0 to 25.4% (Figure 14 - day 7 release). In all seeds the cell viability on day 7 is higher than that observed on day 1, which is to be expected due to the lower amount of IRN released on day 7 (c.f. Figs. 11 A & B). These experiments demonstrate that the drug retains its cytotoxicity during formulation, storage and release.

For the samples derived from the release experiments using Example 4 seeds (Figure 15), it can been seen that the PLGA '5002' sample is not efficacious in killing the GBM cells at either day 1 or day 7, which is not surprising given the low drug release profile seen for '5002' in Figure 12. PLGA `5010', on the other hand, provides a large burst of IRN HCl on day 1 (see Figure 12), however, due to the almost exhaustion of drug release come day 7, `5010' releases less IRN HCl on day 7 than '5004'. The optimal drug release profile over the 7 day period was shown by the '5004' seeds, resulting in less than 30% cell viability with both the day 1 and day 7 release. For this reason, the preferred PLGA is a 50:50 lactide:glycolide PLGA with ester end-capping, having an inherent viscosity of 0.32-0.48 dL/g (`5004' PLGA).

### Example 11: Ex vivo testing of the 2 × 3 mm seeds on primary GBM cells taken from the resection margin of a GBM patient

GBM cells were extracted from tissue taken from the resection margin of GBM patients, cultured and seeded onto 6 well plates. Steam-sterilised 3 mm long PLGA seeds were placed directly onto the GBM cells to mimic the clinical scenario. 3mL of media was used to cover the implants. The media was changed every day to represent the turnover of cerebral spinal fluid as well as the removal of IRN via diffusion, metabolic elimination and permeation into nearby vasculature, which will reduce the concentration of IRN at the resection margin. The study was designed so that MTT assays could be performed on the cells at days 1, 2, 3, 4, 5 and 7.

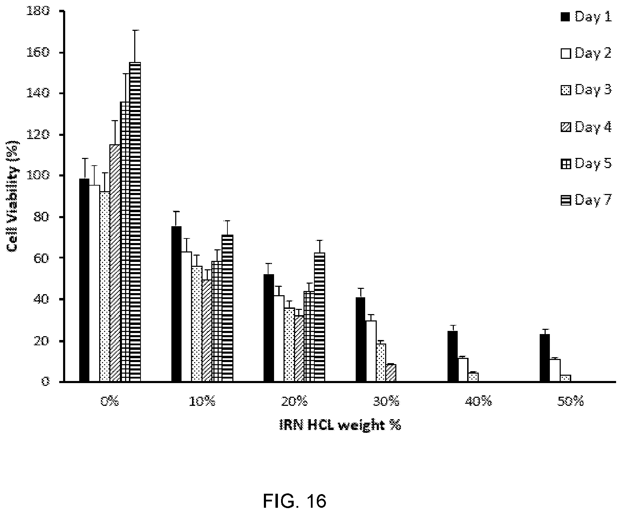

Figure 16 shows that all of the seed implants tested reduced the tumour cell viability with the reduction in cell viability being proportional to the loading of IRN HCL in the seed. The 10 and 20 weight % IRN HCl seeds reduced the cell viability to 49.3 and 32.1% by day 4 respectively; however, significant cell regrowth (representing recurrence) occurred at day 5 and 7. The 30 weight % IRN HCL seeds reduced cell viability to 0% by day 5, while the 40 and 50 weight % IRN HCL seeds achieved this by day 4. Furthermore with the 30, 40 and 50 weight % seeds there was no sign of cell regrowth (recurrence), which suggests that all of the GBM cells were killed off.

These results indicate that the 30 wt%, 40 wt% and 50 wt% seeds deliver sufficient irinotecan over a 7 day period to completely kill off the entire residual tumour cells remaining after resection surgery.

### Example 12: In vivo evaluation of the toxicity of the 30, 40 and 50% w/w seeds in sham resection cavities of non-tumour bearing mice

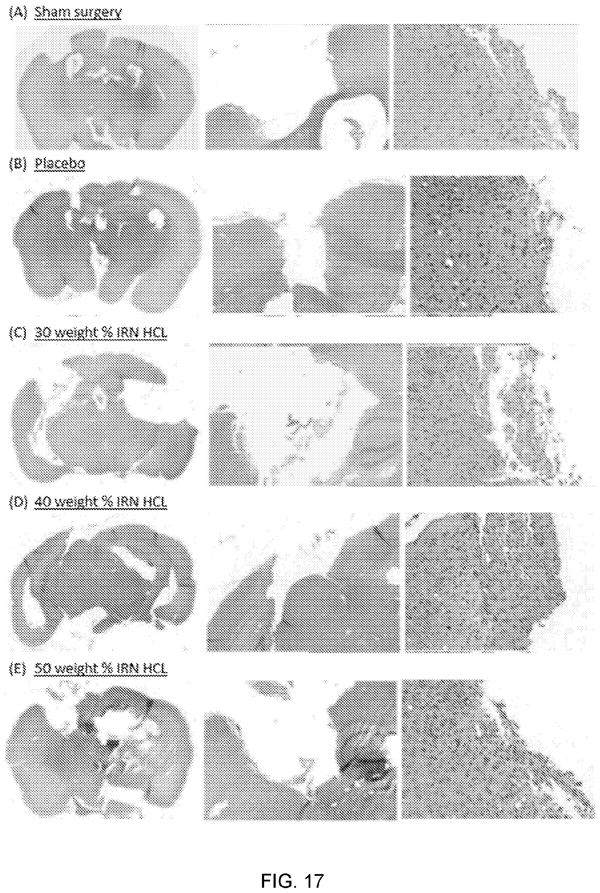

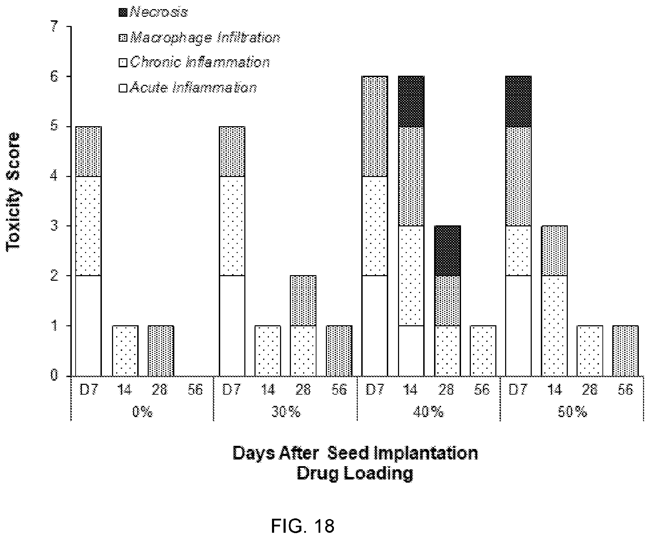

To assess their toxicity, 2 × 2 mm seed formulations with 0, 30, 40 and 50% w/w IRN loadings were implanted into sham resection cavities of non-tumour bearing immunocompetent C57/BL6 mice. Mice were sacrificed via transcardial perfusion at 7, 14, 28, and 56-days post-implant. Brains were stored in 10% formalin upon collection, then moved to 2.5% formalin after 24 hr. Brains were cut in coronal orientation at the rostral and caudal edges of the resection cavity, then embedded in paraffin blocks. Blocks were sectioned into 4 µm thick slices and stained with H&E. The resulting histological slides (Figure 17) were examined by a blinded clinical pathologist. Degree of acute inflammation, chronic inflammation, macrophage infiltration, and necrosis were individually scored on a scale of 0-2 (low, medium, high) and summed to achieve a 'toxicity score' for each seed formulation at each time point (Figure 18). The *in vivo* toxicity study adhered to the NIH Guide for the Care and Use of Laboratory Animals.

The toxicity results presented in Figure 18 shed light on the toxicity profile of the seeds relative to a placebo control. Regardless of IRN loading, moderate levels of acute inflammation are present one week after implantation, however, this inflammation subsides over time. This behaviour is attributable to the wound healing response to resection-induced surgical brain injury. Chronic inflammation due to persistent drug release from the seeds is likewise highest at the onset of implantation as a result of the initial burst of IRN from the seeds and is generally resolved over time, with the exception of 40% which still has detectable levels on day 56. While only mild chronic inflammation is observed in the 0% and 30% groups, moderate inflammation is seen on day 14 in both the 40% and 50% groups, meaning these higher concentrations of IRN elicit a more intense inflammatory response. This observation is further supported by the presence of necrosis in both the 40% and 50% groups. In summary, the 30% seeds do not appear any more toxic than the placebo control in any substantive way, whereas the 40% and 50% seeds cause elevated immune activity and temporary damage to parenchymal tissue.

### Example 13: In vivo testing of the 30, 40 and 50% w/w seeds in a GBM mouse resection model

The U-87MG human GBM cell line has been characterized as a glial tumour with the histopathological appearance of a GBM. The cell line was obtained from ATCC, Manassas, Virginia, USA. The GBM cell line was maintained in culture in DMEM media in an incubator with 5% CO2 at 37°C. Flasks were kept in the logarithmic growth phase and cells were passaged every 3-4 days.

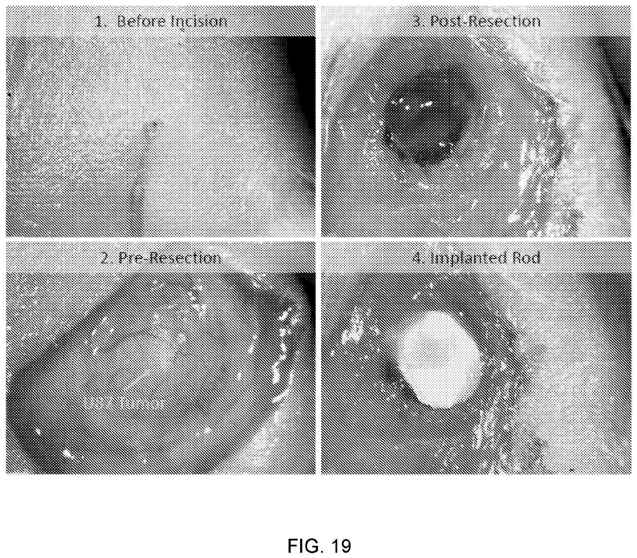

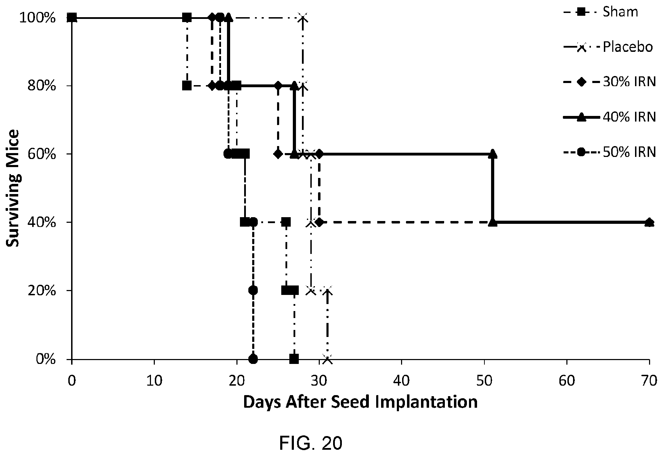

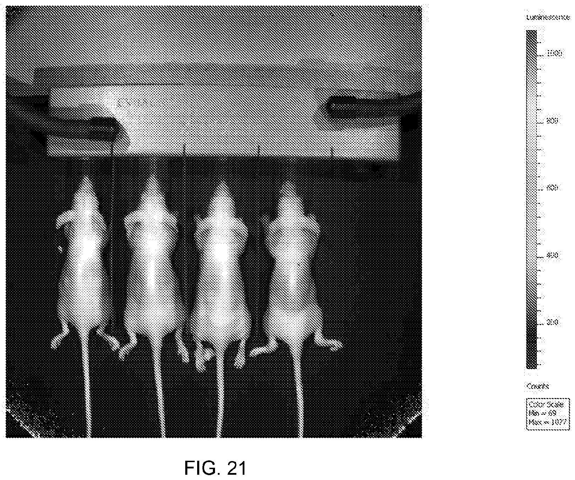

Orthotopic GBM tumours were established in the brains of immunodeficient athymic nude mice (n = 5 in each group) via stereotaxic injection as previously described [Shah et al., J. Neurosci. (2008), 28, 4406-4413; van Eekelen et al., Oncogene (2010), 29, 3185-3195]. Briefly, 1 × 10⁵ U87 mCherry-Fluc (U87 mChFI) cells in 3 µl serum-free DMEM were loaded into a 10 µl capacity Hamilton syringe. The needle was positioned at stereotaxic coordinates [3.0, -0.5, -1.0] from the bregma point. Tumour cells were then injected at 1 µl/min, allowed to settle for 5 min, then the needle was retracted at 0.5 mm/min. The tumours were given one week to engraft and grow. Established tumours were then resected under fluorescent guidance, and then 2 × 2 mm seeds with either 30, 40 and 50 wt% IRN HCl loadings were implanted into the resulting resection cavity (Figure 19). The mice were monitored for survival over 70 days and Kaplan-Meier survival curves produced (Figure 20). Changes in tumour volume were tracked by bioluminescence. Mice were injected with 150 mg/kg luciferin IP, and then imaged 10 min later in an IVIS Kinetic imager under isoflurane anesthesia. Identically-sized regions of interest were drawn over the heads of each mouse, and average radiance was recorded (Figure 21 - imaging of surviving mice at day 70 post implantation). The *in vivo* efficacy study adhered to the NIH Guide for the Care and Use of Laboratory Animals.

The efficacy data (Figure 20) demonstrates that 100% of the mice in the sham (no implant) and placebo groups had died by day 27 and 31 respectively. However, 100% of the mice in the 50 wt% implant group died by day 22, which is due to the level of toxicity observed in the toxicity study for this drug loading (Example 12). The mice in the 30 wt% and 40 wt % implant groups showed long-term survival with 40% of the mice still alive at day 70. Furthermore, the surviving mice show no expected symptoms associated with a recurring brain tumour and when imaged using Fluc bioluminescence imaging none of them showed any sign of brain fluorescence, which would have been indicative of tumour recurrence (Fig. 21). The *in vivo* testing indicates that the preferred drug loadings in terms of a balance between tumour cytotoxicity and systemic toxicity are the 30 wt% and 40 wt % IRN drug loadings.

### Example 14: In vitro release of IRN from PLGA seeds prepared by HME and Compression

10%, 30% and 50% w/w IRN HCl-loaded seeds were prepared by both hot melt extrusion (HME) and compression as described in Example 7.

Individual seeds were placed into a sealed flask containing 5 mL of phosphate buffered (pH 7.4) solution (sink conditioned media) and placed into an orbital shaking incubator (Unitron HT infors) at 37 °C and 60 rpm. Complete media replacement was performed at 1, 2, 3, 4, and 7 days. The samples where filtered using a 0.45µm filter and analysed for IRN content using the irinotecan HPLC method described above.

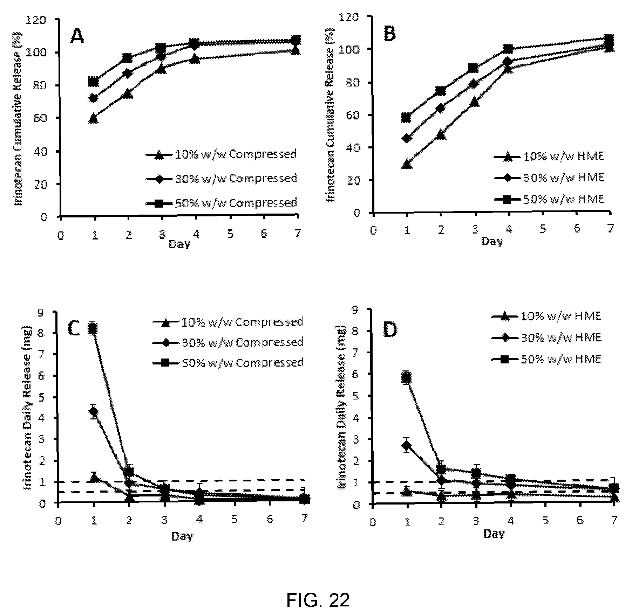

Figure 22 shows the measured *in vitro* release profiles. The seeds prepared by compression (Figs. 22 A & C) delivered a very large 'initial burst' of IRN over the first 24 hours and by day 4 almost all the IRN had been released, with very limited release of IRN between days 4 and 7 (Fig. 22C). The seeds prepared by HME (Figs. 22 B & D) delivered an 'initial burst' release over the first 24 hours, followed by a prolonged period of slower release until day 7. The 30% and 50% w/w HME seeds were capable of releasing at least 500 to 1000 µg of IRN per day for the full 7 days (as represented by the lower and upper dotted lines in Figure 22D) indicating coverage matching the desired therapeutic window for the treatment of gliomas. In this regard, HME is superior to compression in delivering IRN-loaded seeds with the desired properties for treating solid tumours.

### Example 15: Determination of the GBM cell cytotoxicity of IRN, temozolomide (TMZ) and IRN in combination with pitavastatin (PVT)

TMZ, IRN and IRN + PVT were added at increasing concentrations up to 5 log nm to cultured primary GBM cells taken from eight patients with recurrent GBM. After 5 days of exposure an MTT assay was performed to assess % cell viability.

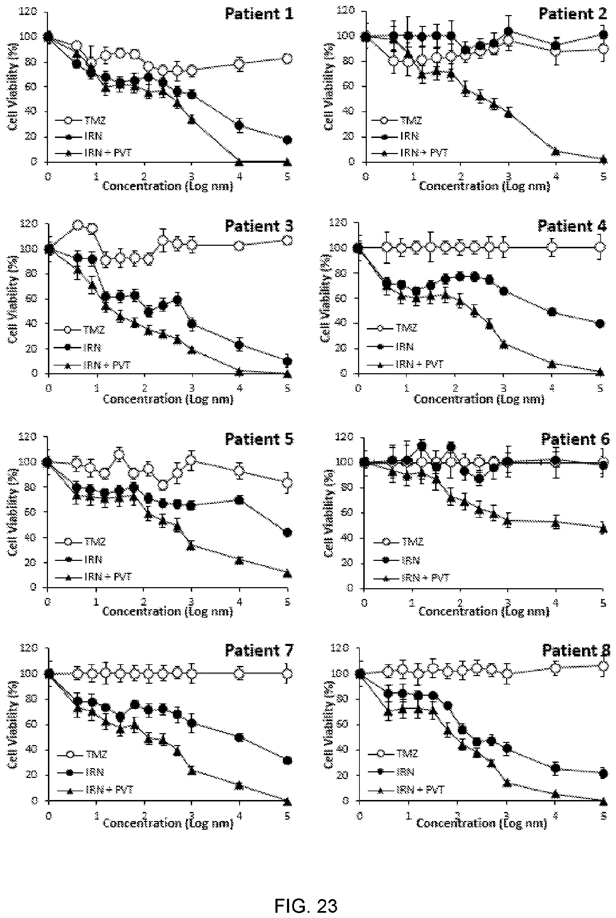

Figure 23 shows the cytotoxicity of the three treatments. All eight patient cell lines evaluated did not respond significantly to TMZ treatment. Six of the eight patient cell lines responded to treatment with IRN and all eight patient cell lines responded to treatment with IRN + PVT. In the combination arm, at a 5 log nm concentration of both IRN and PVT there were no viable GBM cells in six of the eight patient lines.

In a separate experiment, 5 log nm TMZ, 3.5 log nm IRN or 2.5 log nm IRN + PVT (the exposure concentrations used were based on the average IC50 values determined from Figure 23 - as temozolomide did not produce an IC50, a value of 5.0 Log nM was used) were added to cultured primary GBM cells taken from four patients with recurrent GBM. Average % cell viability was assessed by MTT assay after 3, 5, 7, 9 and 11 days exposure.

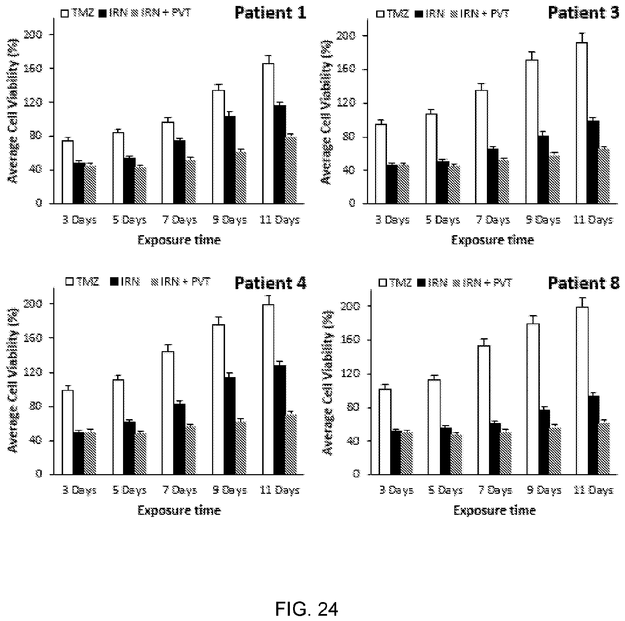

Figure 24 shows the cytotoxicity of the three treatments over the differing exposure times. In all three treatment arms for all four patient cell lines, the cell viability was lowest after 3 days, with viability increasing out to 11 days. In the TMZ arm the cell viability was between 160 and 200%, while in the IRN alone arm, cell viability was between 80 and 120%. However, in the IRN + PVT arm, the cell viability at day 11 was between 60 and 80%. This data suggests that the IRN + PVT combination is better at slowing down recurrence compared to TMZ or IRN alone.

### Example 16: In vitro release of IRN & PVT from dual-layered PLGA seeds

IRN-HCl (30% w/w) and PVT (50% w/w) loaded dual-layer seeds were prepared according to Example 6A and IRN-HCl (40% w/w) and PVT (50% w/w) loaded dual-layer seeds were prepared according to Example 6B.

Individual seeds were placed into a sealed flask containing 5 mL of phosphate buffered (pH 7.4) solution (sink conditioned media) and placed into an orbital shaking incubator (Unitron HT infors) at 37 °C and 60 rpm. Complete media replacement was performed every day for 14 days. The samples where filtered using a 0.45µm filter and analysed for IRN content and PVT content over 14 days using the irinotecan HPLC method described above.

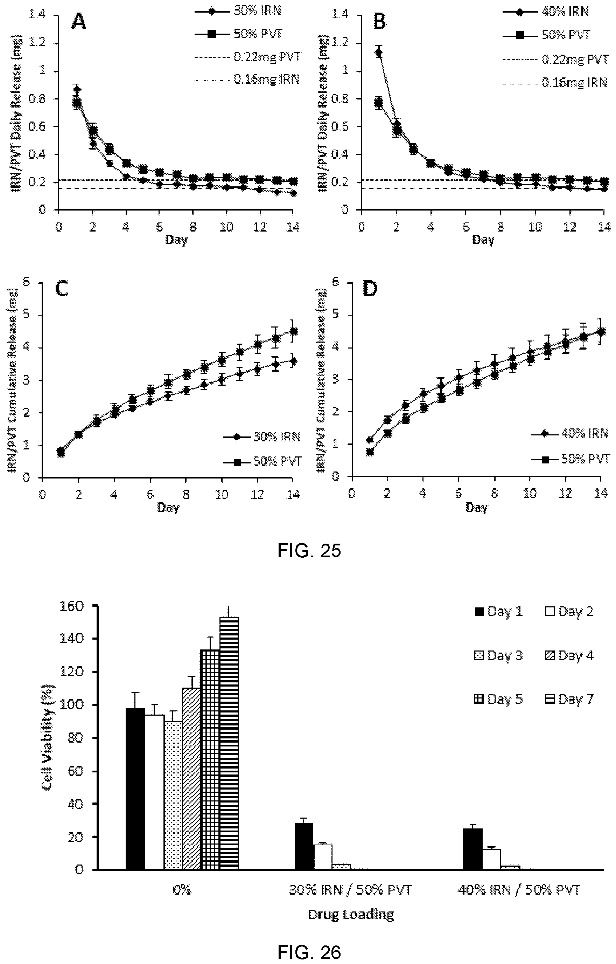

Figure 25 shows the measured *in vitro* release profiles as both daily (Figs. 25A & 25B) and cumulative (Figs. 25C & 25D) API release. The dual-layer seeds gave an initial burst of both IRN and PVT over the first 2-4 days, followed by a sustained release of API out to 14 days. The dashed lines in Figs. 25A & 25B represent the potential daily amounts of IRN and PVT (0.16 mg and 0.22 mg respectively) that need to be released in order to be effective based on the cytotoxicity data from Example 15 (Figures 23 and 24). It was observed that both seeds (30:50% w/w and 40:50% w/w IRN:PVT) released at least these amounts of both APIs continuously over at least 12 days.

### Example 17: Ex vivo testing of IRN & PVT-loaded dual-layered PLGA seeds on primary GBM cells taken from the resection margin of a GBM patient

GBM cells were extracted from tissue taken from the resection margin of a recurrent GBM patient, cultured and seeded onto 6 well plates. Steam-sterilised seeds [(A) 2 mm × 6 mm PLGA seeds with no API added - control; (B) IRN-HCl (30% w/w) and PVT (50% w/w) loaded dual-layer seeds prepared according to Example 6A; and (C) IRN-HCl (40% w/w) and PVT (50% w/w) loaded dual-layer seeds prepared according to Example 6B] were placed directly onto the GBM cells to mimic the clinical scenario. 3mL of media was used to cover the implants. The media was changed every day to represent the turnover of cerebral spinal fluid as well as the removal of API via diffusion, metabolic elimination and permeation into nearby vasculature, which will reduce the concentration of API at the resection margin. The study was designed so that MTT assays could be performed on the cells at days 1, 2, 3, 4, 5 and 7.

Figure 26 shows that both the seed implants tested reduced the tumour cell viability drastically compared to the control seed implant. Both dual-layer IRN/PVT seed implants demonstrated similar ex *vivo* GBM cell cytotoxicity, with negligible cell viability being seen after 4 days incubation. There was no sign of cell regrowth (recurrence) out to 7 days post implant, which suggests that all of the GBM cells were killed off by the IRN & PVT combination seeds.

## Claims

1. A chemotherapeutic seed comprising a biodegradable polymer and a drug selected from irinotecan, a pharmaceutically acceptable salt of irinotecan, 7-ethyl-10-hydroxy-camptothecin (SN-38), and a pharmaceutically acceptable salt of SN-38; wherein the seed has a primary length of at least 0.5 mm, and wherein primary length refers to the shortest dimension of the seed.

2. A seed according to claim 1, wherein the biodegradable polymer is (i) poly(lactic-co-glycolic acid), optionally end-capped with ester groups; or (ii) polylactic acid.

3. A seed according to claim 1 or claim 2, wherein the biodegradable polymer has an acid number less than or equal to 1 mg KOH/g.

4. A seed according to claims 2 to 3, wherein the poly(lactic-co-glycolic acid) has:
a) a lactic acid:glycolic acid weight ratio of 5:95, 15:85, 25:75, 40:60, 50:50, 60:40, 75:25, 85:15, or 95:5; and/or.
b) an inherent viscosity of 0.15 to 1.2 dL/g, when measured at 25 °C in chloroform at a concentration of 0.5 g/dL.

5. A seed according to any one of the preceding claims, wherein the drug is present in a loading of 1 to 50 weight % relative to the weight of the seed, such as 25 to 45 weight % relative to the weight of the seed.

6. A seed according to any one of the preceding claims, wherein the drug is a pharmaceutically acceptable salt of irinotecan, such as irinotecan hydrochloride.

7. A seed according to any one of the preceding claims, wherein the seed has a primary length of 1 to 4 mm; and/or a secondary length of 2 to 6 mm.

8. A seed according to claim 7, wherein the seed is substantially cylindrically-shaped, the primary length is the seed diameter and the secondary length is the seed length; and optionally wherein the seed has a primary length of 2 mm and a secondary length of 3 mm or 6 mm.

9. A seed according to any one of the preceding claims, wherein the seed further comprises a pharmaceutically acceptable plasticiser; and optionally wherein the plasticiser is selected from poloxamers, polyethylene glycols, polyvinyl acetate and macrogolglycerol hydroxystearate.

10. A seed according to any one claims 1 to 9, wherein the seed does not contain a plasticiser.

11. A seed according to any one of the preceding claims, wherein the seed when placed in an aqueous physiological-type environment:
a) continuously releases the drug over a period of at least 3 days, at least 4 days, at least 5 days, at least 6 days or at least 7 days;
b) releases:
i. more than 20% drug after 1 day;
ii. more than 30% drug after 3 days;
iii. more than 40% drug after 5 days; and/or
iv. more than 50% drug after 7 days;
relative to the total amount of drug released; and/or
c) releases at least 300 to 1000 µg of drug per day for a period of at least 5 days, at least 6 days or at least 7 days.

12. A seed according to any one of claims 1 to 11, the seed further comprising one or more additional therapeutic agents selected from anti-cancer agents (such as bevacizumab), statins (such as pitavastatin), antiplatelet agents (such as ticlopidine), antifungal agents (such as itraconazole), angiotensin-converting enzyme (ACE) inhibitors (such as captopril), enzyme acetaldehyde dehydrogenase inhibitors (such as disulfiram), anti-inflammatory agents (such as celecoxib) and copper gluconate; and optionally wherein the seed comprises two layers and wherein the first layer comprises a biodegradable polymer and a drug selected from irinotecan, a pharmaceutically acceptable salt of irinotecan, SN-38, and a pharmaceutically acceptable salt of SN-38; and the second layer comprises a biodegradable polymer and an additional therapeutic agent (such as pitavastatin).

13. A pharmaceutical composition comprising one or more seeds according to claims 1 to 12.

14. A seed according to any one of claims 1 to 12, or a pharmaceutical composition according to claim 13, for use in therapy, such as in the treatment of a brain tumour.

15. A process for the preparation of a chemotherapeutic seed according to any one of claims 1 to 12, the process comprising the steps of:
a) charging the drug and biodegradable polymer to a hot melt extruder;
b) extruding the mixture from the extruder;
c) forming the extrudate from step b) into one or more seeds of the required dimensions.

## Patentansprüche

1. Chemotherapeutisches Seed, umfassend ein biologisch abbaubares Polymer und ein Arzneimittel, das aus Irinotecan, einem pharmazeutisch unbedenklichen Salz von Irinotecan, 7-Ethyl-10-hydroxycamptothecin (SN-38) und einem pharmazeutisch unbedenklichen Salz von SN-38 ausgewählt ist; wobei das Seed eine Primärlänge von mindestens 0,5 mm aufweist und wobei sich die Primärlänge auf die kürzeste Abmessung des Seeds bezieht.

2. Seed nach Anspruch 1, wobei das biologisch abbaubare Polymer (i) Poly(milch-co-glykolsäure), optional mit Estergruppen endverschlossen, oder (ii) Polymilchsäure ist.

3. Seed nach Anspruch 1 oder Anspruch 2, wobei das biologisch abbaubare Polymer eine Säurezahl von kleiner oder gleich 1 mg KOH/g aufweist.

4. Seed nach den Ansprüchen 2 bis 3, wobei die Poly(milchsäure-co-glykolsäure) Folgendes aufweist:
a) ein Gewichtsverhältnis von Milchsäure:Glykolsäure von 5:95, 15:85, 25:75, 40:60, 50:50, 60:40, 75:25, 85:15 oder 95:5; und/oder
b) eine inhärente Viskosität von 0,15 bis 1,2 dl/g, gemessen bei 25 °C in Chloroform bei einer Konzentration von 0,5 g/dl.

5. Seed nach einem der vorhergehenden Ansprüche, wobei das Arzneimittel in einer Beladung von 1 bis 50 Gewichts-%, bezogen auf das Gewicht des Seeds, vorhanden ist, wie 25 bis 45 Gewichts-%, bezogen auf das Gewicht des Seeds.

6. Seed nach einem der vorhergehenden Ansprüche, wobei das Arzneimittel ein pharmazeutisch unbedenkliches Salz von Irinotecan, wie Irinotecanhydrochlorid, ist.

7. Seed nach einem der vorhergehenden Ansprüche, wobei das Seed eine Primärlänge von 1 bis 4 mm aufweist; und/oder eine Sekundärlänge von 2 bis 6 mm.

8. Seed nach Anspruch 7, wobei das Seed im Wesentlichen zylindrisch geformt ist, die Primärlänge der Seed-Durchmesser und die Sekundärlänge die Seed-Länge ist; und wobei das Seed optional eine Primärlänge von 2 mm und eine Sekundärlänge von 3 mm oder 6 mm aufweist.

9. Seed nach einem der vorhergehenden Ansprüche, wobei das Seed ferner einen pharmazeutisch unbedenklichen Weichmacher umfasst, und wobei der Weichmacher optional aus Poloxameren, Polyethylenglykolen, Polyvinylacetat und Macrogolglycerolhydroxystearat ausgewählt ist.

10. Seed nach einem der Ansprüche 1 bis 9, wobei das Seed keinen Weichmacher enthält.

11. Seed nach einem der vorhergehenden Ansprüche, wobei das Seed, wenn es in eine wässrige Umgebung des physiologischen Typs gegeben wird:
a) das Arzneimittel kontinuierlich über einen Zeitraum von mindestens 3 Tagen, mindestens 4 Tagen, mindestens 5 Tagen, mindestens 6 Tagen oder mindestens 7 Tagen freisetzt;
b) das Arzneimittel wie folgt freisetzt:
i. mehr als 20 % des Arzneimittels nach 1 Tag;
ii. mehr als 30 % des Arzneimittels nach 3 Tagen,
iii. mehr als 40 % des Arzneimittels nach 5 Tagen, und/oder
iv. mehr als 50 % des Arzneimittels nach 7 Tagen,
im Verhältnis zur Gesamtmenge des freigesetzten Arzneimittels; und/oder
c) mindestens 300 bis 1000 µg des Arzneimittels pro Tag über einen Zeitraum von mindestens 5 Tagen, mindestens 6 Tagen oder mindestens 7 Tagen freisetzt.

12. Seed nach einem der Ansprüche 1 bis 11, wobei das Seed ferner ein oder mehrere zusätzliche therapeutische Mittel umfasst, die aus Antikrebsmitteln (wie Bevacizumab), Statinen (wie Pitavastatin), Thrombozytenaggregationshemmern (wie Ticlopidin), Antimykotika (wie Itraconazol), Angiotensin-Converting-Enzym(ACE)-Hemmern (wie Captopril), Hemmern des Enzyms Acetaldehyd-Dehydrogenase (wie Disulfiram), entzündungshemmenden Mitteln (wie Celecoxib) und Kupfergluconat ausgewählt sind; und wobei das Seed optional zwei Schichten umfasst und wobei die erste Schicht ein biologisch abbaubares Polymer und ein Arzneimittel umfasst, das aus Irinotecan, einem pharmazeutisch unbedenklichen Salz von Irinotecan, SN-38 und einem pharmazeutisch unbedenklichen Salz von SN-38 ausgewählt ist, und die zweite Schicht ein biologisch abbaubares Polymer und ein zusätzliches therapeutisches Mittel (wie Pitavastatin) umfasst.

13. Pharmazeutische Zusammensetzung, einen oder mehrere Seeds nach den Ansprüchen 1 bis 12 umfassend.

14. Seed nach einem der Ansprüche 1 bis 12 oder pharmazeutische Zusammensetzung nach Anspruch 13 zur Verwendung bei der Therapie, wie bei der Behandlung eines Hirntumors.

15. Verfahren zur Herstellung eines chemotherapeutischen Seeds nach einem der Ansprüche 1 bis 12, wobei das Verfahren die folgenden Schritte umfasst:
a) Laden des Arzneimittels und des biologisch abbaubaren Polymers in einen Heißschmelzextruder;
b) Extrudieren der Mischung aus dem Extruder;
c) Formen des Extrudats aus Schritt b) zu einem oder mehreren Seeds der erforderlichen Abmessungen.

## Revendications

1. Grain chimiothérapeutique comprenant un polymère biodégradable et un médicament sélectionné parmi irinotécan, un sel de qualité pharmaceutique de l'irinotécan, 7-éthyl-10-hydroxy-camptothécine (SN-38), et un sel de qualité pharmaceutique de SN-38 ; le grain comportant une longueur primaire d'au moins 0,5 mm, et la longueur primaire désignant la dimension la plus courte du grain.

2. Grain selon la revendication 1, dans lequel le polymère biodégradable est (i) poly(acide lactique-co-glycolique), éventuellement coiffé aux extrémités par des groupes ester ; ou (ii) acide polylactique.

3. Grain selon la revendication 1 ou la revendication 2, dans lequel le polymère biodégradable comporte un indice d'acidité inférieur ou égal à 1 mg KOH/g.

4. Grain selon les revendications 2 à 3, dans lequel le poly(acide lactique-co-glycolique) comporte :
a) un rapport de poids d'acide lactique:acide glycolique de 5:95, 15:85, 25:75, 40:60, 50:50, 60:40, 75:25, 85:15, ou 95:5 ; et/ou
b) une viscosité inhérente de 0,15 à 1,2 dL/g, mesurée à 25 °C dans du chloroforme à une concentration de 0,5 g/dL.

5. Grain selon l'une quelconque des revendications précédentes, dans lequel le médicament est présent dans un chargement de 1 à 50 % en poids par rapport au poids du grain, par exemple 25 à 45 % en poids par rapport au poids du grain.

6. Grain selon l'une quelconque des revendications précédentes, dans lequel le médicament est un sel de qualité pharmaceutique de l'irinotécan, tel que l'hydrochlorure d'irinotécan.

7. Grain selon l'une quelconque des revendications précédentes, le grain comportant une longueur primaire de 1 à 4 mm ; et/ou une longueur secondaire de 2 à 6 mm.

8. Grain selon la revendication 7, le grain étant de forme substantiellement cylindrique, la longueur primaire étant le diamètre du grain et la longueur secondaire étant la longueur du grain ; et éventuellement le grain comportant une longueur primaire de 2 mm et une longueur secondaire de 3 mm ou 6 mm.

9. Grain selon l'une quelconque des revendications précédentes, le grain comprenant en outre un plastifiant de qualité pharmaceutique ; et le plastifiant étant éventuellement sélectionné parmi : poloxamères, polyéthylèneglycols, acétate de polyvinyle et macrogolglycérol-hydroxystéarate.

10. Grain selon l'une quelconque des revendications 1 à 9, le grain ne contenant pas de plastifiant.

11. Grain selon l'une quelconque des revendications précédentes où, lorsque le grain est placé dans un milieu aqueux de type physiologique :
a) il libère continuellement le médicament au cours d'une période d'au moins 3 jours, au moins 4 j ours, au moins 5 j ours, au moins 6 j ours ou au moins 7 jours ;
b) il libère :
i. plus de 20 % du médicament après 1 jour ;
ii. plus de 30 % du médicament après 3 jours ;
iii. plus de 40 % du médicament après 5 jours ; et/ou
iv. plus de 50 % du médicament après 7 jours ;
par rapport à la quantité totale de médicament libérée ; et/ou
c) il libère au moins 300 à 1000 µg de médicament par jour pendant une période d'au moins 5 jours, au moins 6 jours ou au moins 7 jours.

12. Grain selon l'une quelconque des revendications 1 à 11, le grain comprenant en outre un ou plusieurs agents thérapeutiques sélectionnés parmi des agents anti-cancer (tels que bévacizumab), statines (telles que pitavastatine), anti-agrégants plaquettaires (tels que ticlopidine), agents antifongiques (tels qu'itraconazole), inhibiteurs de l'enzyme de conversion de l'angiotensine (ECA) (tels que captopril), inhibiteurs de l'enzyme acétaldéhyde déshydrogénase (tels que disulfirame), agents anti-inflammatoires (tels que célécoxib) et gluconate de cuivre ; et éventuellement, le grain comprenant deux couches et dont la première couche comprend un polymère biodégradable et un médicament sélectionné parmi irinotécan, un sel de qualité pharmaceutique d'irinotécan, SN-38, et un sel de qualité pharmaceutique de SN-38 ; et la deuxième couche comprend un polymère biodégradable et un agent thérapeutique supplémentaire (tel que pitavastatine).

13. Composition pharmaceutique comprenant un ou plusieurs grains selon les revendications 1 à 12.

14. Grain selon l'une quelconque des revendications 1 à 12, ou composition pharmaceutique selon la revendication 13, destiné à être utilisé dans une thérapie, telle que le traitement d'une tumeur cérébrale.

15. Procédé pour la préparation d'un grain chimiothérapeutique selon l'une quelconque des revendications 1 à 12, le procédé comprenant les étapes de :
a) chargement du médicament et du polymère biodégradable dans une extrudeuse à thermofusion ;
b) extrusion du mélange contenu dans l'extrudeuse ;
c) formage de l'extrudat obtenu à l'étape b) en un ou plusieurs grains des dimensions requises.
